# FASCICULE DE BREVET EUROPEEN

(11) **EP 2 828 285 B1**
(45) Date de publication et mention de la délivrance du brevet: **25.04.2018**
(21) Numéro de dépôt: 13720081.2
(22) Date de dépôt: 19.03.2013
(51) Int. Cl.: C07K 14/34, A61K 38/16, C12N 15/31, G01N 33/574, C12N 1/21

(54) **INHIBITEUR DE L'HB-EGF DÉRIVÉ DU DOMAINE R DE LA TOXINE DIPHTÉRIQUE POUR LE TRAITEMENT DES MALADIES ASSOCIÉES A L'ACTIVATION DE LA VOIE HB-EGF/EGFR**
HB-EGF INHIBITOR AUS DER DIPHTERIE TOXIN R DOMÄNE ZUR BEHANDLUNG VON KRANKHEITEN DIE ASSOZIIERT SIND MIT DER AKTIVIERUNG DES HB-EGF/EGFR SIGNALWEGS.
HB-EGF INHIBITOR DERIVED FROM DIPHTHERIA TOXIN R DOMAIN FOR THE TREATMENT OF DISEASES ASSOCIATED WITH THE ACTIVATION OF HB-EGF/EGFR PATHWAY.

(30) Priorité: 20.03.2012 FR 1252497
(43) Date de publication de la demande: 28.01.2015
(73) Titulaire: Commissariat à l'Énergie Atomique et aux Énergies Alternatives, 75015 Paris (FR)
(72) Inventeur: GILLET, Daniel, 92160 Antony (FR); VILLIERS, Benoit, F-86120 Roiffe (FR); PICHARD, Sylvain, F-91310 Leuville Sur Orge (FR); MAILLERE, Bernard, F-78000 Versailles (FR); SANSON, Alain, F-91940 Gometz Le Chatel (FR)
(74) Mandataire: Cabinet Plasseraud
(86) Numéro de dépôt international: PCT/IB2013/052173
(87) Numéro de publication internationale: WO 2013/140335

(56) Documents cités:
- EP-A1- 1 894 575
- WO-A1-93/21769
- WO-A1-95/33481
- WO-A2-00/48638
- LOBECK KARIN ET AL: "Towards a recombinant vaccine against diphtheria toxin", INFECTION AND IMMUNITY, vol. 66, no. 2, février 1998 (1998-02), pages 418-423, XP002687264, ISSN: 0019-9567 cité dans la demande
- SHEN W H ET AL: "Participation of lysine 516 and phenylalanine 530 of diphtheria toxin in receptor recognition", JOURNAL OF BIOLOGICAL CHEMISTRY, AMERICAN SOCIETY FOR BIOCHEMISTRY AND MOLECULAR BIOLOGY, US, vol. 269, no. 46, 18 novembre 1994 (1994-11-18), pages 29077-29084, XP002153861, ISSN: 0021-9258 cité dans la demande
- BUZZI SILVIO ET AL: "CRM197 (nontoxic diphtheria toxin): effects on advanced cancer patients", CANCER IMMUNOLOGY AND IMMUNOTHERAPY, SPRINGER-VERLAG, BERLIN, DE, vol. 53, no. 11, 1 novembre 2004 (2004-11-01), pages 1041-1048, XP002475696, ISSN: 0340-7004, DOI: 10.1007/S00262-004-0546-4
- LOUIE GORDON V ET AL: "Crystal structure of the complex of diphtheria toxin with an extracellular fragment of its receptor", MOLECULAR CELL, vol. 1, no. 1, décembre 1997 (1997-12), pages 67-78, XP002687267, ISSN: 1097-2765 cité dans la demande
- MIYAZONO KOHEI: "Ectodomain shedding of HB-EGF: A potential target for cancer therapy", JOURNAL OF BIOCHEMISTRY (TOKYO), vol. 151, no. 1, janvier 2012 (2012-01), pages 1-3 URL, XP002687266,
- VILLIERS BENOIT ET AL: "Engineering of an HB-EGF Inhibitor from the Diphtheria Toxin Receptor-Binding Domain for the Treatment of HB-EGF-Related Diseases", TOXICON, vol. 60, no. 2, Sp. Iss. SI, août 2012 (2012-08), pages 106-107, XP002687265, & 17TH WORLD CONGRESS OF THE INTERNATIONAL-SOCIETY-ON-TOXINOLOGY (IST)/VENOM WEEK/4TH INTERNATIONAL SC; HONOLULU, HI, USA; JULY 08 -13, 2012 ISSN: 0041-0101

### SEQUENCE LISTING

<110> COMMISSARIAT A L'ENERGIE ATOMIQUE ET AUX ENERGIES ALTERNATIVES
<120> Inhibiteur de l'HB-EGF dérivé du domaine R de la toxine diphtérique pour le traitement des maladies associées à l'activation de la voie HB-EGF/EGFR
<130> 263FR495
<160> 109
<170> PatentIn version 3.5
<210> 1
   <211> 535
   <212> PRT
   <213> Corynebacterium diphtheriae
<400> 1
   Gly Ala Asp Asp Val Val Asp Ser Ser Lys Ser Phe Val Met Glu Asn
   1               5                   10                  15
   Phe Ser Ser Tyr His Gly Thr Lys Pro Gly Tyr Val Asp Ser Ile Gln
               20                  25                  30
   Lys Gly Ile Gln Lys Pro Lys Ser Gly Thr Gln Gly Asn Tyr Asp Asp
           35                  40                  45
   Asp Trp Lys Gly Phe Tyr Ser Thr Asp Asn Lys Tyr Asp Ala Ala Gly
       50                  55                  60
   Tyr Ser Val Asp Asn Glu Asn Pro Leu Ser Gly Lys Ala Gly Gly Val
   65                  70                  75                  80
   Val Lys Val Thr Tyr Pro Gly Leu Thr Lys Val Leu Ala Leu Lys Val
                   85                  90                  95
   Asp Asn Ala Glu Thr Ile Lys Lys Glu Leu Gly Leu Ser Leu Thr Glu
               100                 105                 110
   Pro Leu Met Glu Gln Val Gly Thr Glu Glu Phe Ile Lys Arg Phe Gly
           115                 120                 125
   Asp Gly Ala Ser Arg Val Val Leu Ser Leu Pro Phe Ala Glu Gly Ser
       130                 135                 140
   Ser Ser Val Glu Tyr Ile Asn Asn Trp Glu Gln Ala Lys Ala Leu Ser
   145                 150                 155                 160
   Val Glu Leu Glu Ile Asn Phe Glu Thr Arg Gly Lys Arg Gly Gln Asp
   165 170 175
   Ala Met Tyr Glu Tyr Met Ala Gln Ala Cys Ala Gly Asn Arg Val Arg
   180 185 190
   Arg Ser Val Gly Ser Ser Leu Ser Cys Ile Asn Leu Asp Trp Asp Val
   195 200 205
   Ile Arg Asp Lys Thr Lys Thr Lys Ile Glu Ser Leu Lys Glu His Gly
   210 215 220
   Pro Ile Lys Asn Lys Met Ser Glu Ser Pro Asn Lys Thr Val Ser Glu
   225 230 235 240
   Glu Lys Ala Lys Gln Tyr Leu Glu Glu Phe His Gln Thr Ala Leu Glu
   245 250 255
   His Pro Glu Leu Ser Glu Leu Lys Thr Val Thr Gly Thr Asn Pro Val
   260 265 270
   Phe Ala Gly Ala Asn Tyr Ala Ala Trp Ala Val Asn Val Ala Gln Val
   275 280 285
   Ile Asp Ser Glu Thr Ala Asp Asn Leu Glu Lys Thr Thr Ala Ala Leu
   290 295 300
   Ser Ile Leu Pro Gly Ile Gly Ser Val Met Gly Ile Ala Asp Gly Ala
   305 310 315 320
   Val His His Asn Thr Glu Glu Ile Val Ala Gln Ser Ile Ala Leu Ser
   325 330 335
   Ser Leu Met Val Ala Gln Ala Ile Pro Leu Val Gly Glu Leu Val Asp
   340 345 350
   Ile Gly Phe Ala Ala Tyr Asn Phe Val Glu Ser Ile Ile Asn Leu Phe
   355 360 365
   Gln Val Val His Asn Ser Tyr Asn Arg Pro Ala Tyr Ser Pro Gly His
   370 375 380
   Lys Thr Gln Pro Phe Leu His Asp Gly Tyr Ala Val Ser Trp Asn Thr
   385 390 395 400
   Val Glu Asp Ser Ile Ile Arg Thr Gly Phe Gln Gly Glu Ser Gly His
   405 410 415
   Asp Ile Lys Ile Thr Ala Glu Asn Thr Pro Leu Pro Ile Ala Gly Val
               420                 425                 430
   Leu Leu Pro Thr Ile Pro Gly Lys Leu Asp Val Asn Lys Ser Lys Thr
           435                 440                 445
   His Ile Ser Val Asn Gly Arg Lys Ile Arg Met Arg Cys Arg Ala Ile
       450                 455                 460
   Asp Gly Asp Val Thr Phe Cys Arg Pro Lys Ser Pro Val Tyr Val Gly
   465                 470                 475                 480
   Asn Gly Val His Ala Asn Leu His Val Ala Phe His Arg Ser Ser Ser
                   485                 490                 495
   Glu Lys Ile His Ser Asn Glu Ile Ser Ser Asp Ser Ile Gly Val Leu
               500                 505                 510
   Gly Tyr Gln Lys Thr Val Asp His Thr Lys Val Asn Ser Lys Leu Ser
           515                 520                 525
   Leu Phe Phe Glu Ile Lys Ser
       530                 535
<210> 2
   <211> 158
   <212> PRT
   <213> artificial sequence
<220>
   <223> synthetic protein (DTR variant Y380K/L390T encoded by clone 1D3)
<400> 2
   Met Gly Lys Ser Pro Gly His Lys Thr Gln Pro Phe Thr His Asp Gly
   1               5                   10                  15
   Tyr Ala Val Ser Trp Asn Thr Val Glu Asp Ser Ile Ile Arg Thr Gly
               20                  25                  30
   Phe Gln Gly Glu Ser Gly His Asp Ile Lys Ile Thr Ala Glu Asn Thr
           35                  40                  45
   Pro Leu Pro Ile Ala Gly Val Leu Leu Pro Thr Ile Pro Gly Lys Leu
       50                  55                  60
   Asp Val Asn Lys Ser Lys Thr His Ile Ser Val Asn Gly Arg Lys Ile
   65                  70                  75                  80
   Arg Met Arg Cys Arg Ala Ile Asp Gly Asp Val Thr Phe Cys Arg Pro
                   85                  90                  95
   Lys Ser Pro Val Tyr Val Gly Asn Gly Val His Ala Asn Leu His Val
               100                 105                 110
   Ala Phe His Arg Ser Ser Ser Glu Lys Ile His Ser Asn Glu Ile Ser
           115                 120                 125
   Ser Asp Ser Ile Gly Val Leu Gly Tyr Gln Lys Thr Val Asp His Thr
       130                 135                 140
   Lys Val Asn Ser Lys Leu Ser Leu Phe Phe Glu Ile Lys Ser
   145                 150                 155
<210> 3
   <211> 158
   <212> PRT
   <213> artificial sequence
<220>
   <223> synthetic protein (DTR variant Y380K/Q387E/L390T encoded by clone 1D6)
<400> 3
   Met Gly Lys Ser Pro Gly His Lys Thr Glu Pro Phe Thr His Asp Gly
   1               5                   10                  15
   Tyr Ala Val Ser Trp Asn Thr Val Glu Asp Ser Ile Ile Arg Thr Gly
               20                  25                  30
   Phe Gln Gly Glu Ser Gly His Asp Ile Lys Ile Thr Ala Glu Asn Thr
           35                  40                  45
   Pro Leu Pro Ile Ala Gly Val Leu Leu Pro Thr Ile Pro Gly Lys Leu
       50                  55                  60
   Asp Val Asn Lys Ser Lys Thr His Ile Ser Val Asn Gly Arg Lys Ile
   65                  70                  75                      80
   Arg Met Arg Cys Arg Ala Ile Asp Gly Asp Val Thr Phe Cys Arg Pro
                   85                  90                  95
   Lys Ser Pro Val Tyr Val Gly Asn Gly Val His Ala Asn Leu His Val
               100                 105                 110
   Ala Phe His Arg Ser Ser Ser Glu Lys Ile His Ser Asn Glu Ile Ser
           115                 120                 125
   Ser Asp Ser Ile Gly Val Leu Gly Tyr Gln Lys Thr Val Asp His Thr
           130                 135                 140
   Lys Val Asn Ser Lys Leu Ser Leu Phe Phe Glu Ile Lys Ser
   145                 150                 155
<210> 4
   <211> 158
   <212> PRT
   <213> artificial sequence
<220>
   <223> synthetic protein (DTR variant Y380K/Q387E/P388T/L390T encoded by clone 2C8)
<400> 4
   Met Gly Lys Ser Pro Gly His Lys Thr Glu Thr Phe Thr His Asp Gly
   1               5                   10                  15
   Tyr Ala Val Ser Trp Asn Thr Val Glu Asp Ser Ile Ile Arg Thr Gly
               20                  25                  30
   Phe Gln Gly Glu Ser Gly His Asp Ile Lys Ile Thr Ala Glu Asn Thr
           35                  40                  45
   Pro Leu Pro Ile Ala Gly Val Leu Leu Pro Thr Ile Pro Gly Lys Leu
       50                  55                  60
   Asp Val Asn Lys Ser Lys Thr His Ile Ser Val Asn Gly Arg Lys Ile
   65                  70                  75                  80
   Arg Met Arg Cys Arg Ala Ile Asp Gly Asp Val Thr Phe Cys Arg Pro
                   85                  90                  95
   Lys Ser Pro Val Tyr Val Gly Asn Gly Val His Ala Asn Leu His Val
               100                 105                 110
   Ala Phe His Arg Ser Ser Ser Glu Lys Ile His Ser Asn Glu Ile Ser
           115                 120                 125
   Ser Asp Ser Ile Gly Val Leu Gly Tyr Gln Lys Thr Val Asp His Thr
       130                 135                 140
   Lys Val Asn Ser Lys Leu Ser Leu Phe Phe Glu Ile Lys Ser
   145                 150                 155
<210> 5
   <211> 158
   <212> **PRT**
   <213> artificial sequence
<220>
   <223> synthetic protein (DTR variant Y380K/P382T/Q387E/L390T encoded by clone 2G5)
<400> 5
   Met Gly Lys Ser Thr Gly His Lys Thr Glu Pro Phe Thr His Asp Gly
   1 5 10 15
   Tyr Ala Val Ser Trp Asn Thr Val Glu Asp Ser Ile Ile Arg Thr Gly
   20 25 30
   Phe Gln Gly Glu Ser Gly His Asp Ile Lys Ile Thr Ala Glu Asn Thr
   35 40 45
   Pro Leu Pro Ile Ala Gly Val Leu Leu Pro Thr Ile Pro Gly Lys Leu
   50 55 60
   Asp Val Asn Lys Ser Lys Thr His Ile Ser Val Asn Gly Arg Lys Ile
   65 70 75 80
   Arg Met Arg Cys Arg Ala Ile Asp Gly Asp Val Thr Phe Cys Arg Pro
   85 90 95
   Lys Ser Pro Val Tyr Val Gly Asn Gly Val His Ala Asn Leu His Val
   100 105 110
   Ala Phe His Arg Ser Ser Ser Glu Lys Ile His Ser Asn Glu Ile Ser
   115 120 125
   Ser Asp Ser Ile Gly Val Leu Gly Tyr Gln Lys Thr Val Asp His Thr
   130 135 140
   Lys Val Asn Ser Lys Leu Ser Leu Phe Phe Glu Ile Lys Ser
   145 150 155
<210> 6
   <211> 158
   <212> PRT
   <213> artificial sequence
<220>
   <223> synthetic protein (DTR variant Y380E/P382T/Q387K/L390T encoded by clone 1H3)
<400> 6
   Met Gly Glu Ser Thr Gly His Lys Thr Lys Pro Phe Thr His Asp Gly
   1 5 10 15
   Tyr Ala Val Ser Trp Asn Thr Val Glu Asp Ser Ile Ile Arg Thr Gly
   20 25 30
   Phe Gln Gly Glu Ser Gly His Asp Ile Lys Ile Thr Ala Glu Asn Thr
           35                  40                  45
   Pro Leu Pro Ile Ala Gly Val Leu Leu Pro Thr Ile Pro Gly Lys Leu
       50                  55                  60
   Asp Val Asn Lys Ser Lys Thr His Ile Ser Val Asn Gly Arg Lys Ile
   65                  70                  75                  80
   Arg Met Arg Cys Arg Ala Ile Asp Gly Asp Val Thr Phe Cys Arg Pro
                   85                  90                  95
   Lys Ser Pro Val Tyr Val Gly Asn Gly Val His Ala Asn Leu His Val
               100                 105                 110
   Ala Phe His Arg Ser Ser Ser Glu Lys Ile His Ser Asn Glu Ile Ser
           115                 120                 125
   Ser Asp Ser Ile Gly Val Leu Gly Tyr Gln Lys Thr Val Asp His Thr
       130                 135                 140
   Lys Val Asn Ser Lys Leu Ser Leu Phe Phe Glu Ile Lys Ser
   145                 150                 155
<210> 7
   <211> 158
   <212> PRT
   <213> artificial sequence
<220>
   <223> synthetic protein (DTR variant Y380K/Q387E/L390T/A395T named as DTR1)
<400> 7
   Met Gly Lys Ser Pro Gly His Lys Thr Glu Pro Phe Thr His Asp Gly
   1               5                   10                  15
   Tyr Thr Val Ser Trp Asn Thr Val Glu Asp Ser Ile Ile Arg Thr Gly
               20                  25                  30
   Phe Gln Gly Glu Ser Gly His Asp Ile Lys Ile Thr Ala Glu Asn Thr
           35                  40                  45
   Pro Leu Pro Ile Ala Gly Val Leu Leu Pro Thr Ile Pro Gly Lys Leu
       50                  55                  60
   Asp Val Asn Lys Ser Lys Thr His Ile Ser Val Asn Gly Arg Lys Ile
   65                  70                  75                  80
   Arg Met Arg Cys Arg Ala Ile Asp Gly Asp Val Thr Phe Cys Arg Pro
   85 90 95
   Lys Ser Pro Val Tyr Val Gly Asn Gly Val His Ala Asn Leu His Val
   100 105 110
   Ala Phe His Arg Ser Ser Ser Glu Lys Ile His Ser Asn Glu Ile Ser
   115 120 125
   Ser Asp Ser Ile Gly Val Leu Gly Tyr Gln Lys Thr Val Asp His Thr
   130 135 140
   Lys Val Asn Ser Lys Leu Ser Leu Phe Phe Glu Ile Lys Ser
   145 150 155
<210> 8
   <211> 158
   <212> PRT
   <213> artificial sequence
<220>
   <223> synthetic protein (DTR variant Y380K/Q387E/F389Y/L390T/A395T/G510A named as DTR3)
<400> 8
   Met Gly Lys Ser Pro Gly His Lys Thr Glu Pro Tyr Thr His Asp Gly
   1 5 10 15
   Tyr Thr Val Ser Trp Asn Thr Val Glu Asp Ser Ile Ile Arg Thr Gly
   20 25 30
   Phe Gln Gly Glu Ser Gly His Asp Ile Lys Ile Thr Ala Glu Asn Thr
   35 40 45
   Pro Leu Pro Ile Ala Gly Val Leu Leu Pro Thr Ile Pro Gly Lys Leu
   50 55 60
   Asp Val Asn Lys Ser Lys Thr His Ile Ser Val Asn Gly Arg Lys Ile
   65 70 75 80
   Arg Met Arg Cys Arg Ala Ile Asp Gly Asp Val Thr Phe Cys Arg Pro
   85 90 95
   Lys Ser Pro Val Tyr Val Gly Asn Gly Val His Ala Asn Leu His Val
   100 105 110
   Ala Phe His Arg Ser Ser Ser Glu Lys Ile His Ser Asn Glu Ile Ser
   115 120 125
   Ser Asp Ser Ile Ala Val Leu Gly Tyr Gln Lys Thr Val Asp His Thr
       130                 135                 140
   Lys Val Asn Ser Lys Leu Ser Leu Phe Phe Glu Ile Lys Ser
   145                 150                 155
<210> 9
   <211> 158
   <212> PRT
   <213> artificial sequence
<220>
   <223> synthetic protein (DTRvariant
   Y380K/Q387E/F389Y/L390T/A395T/N399K/V452T/V483Q/H492E/S494K/G510A
   /T517E named as DTR8)
<400> 9
   Met Gly Lys Ser Pro Gly His Lys Thr Glu Pro Tyr Thr His Asp Gly
   1               5                   10                  15
   Tyr Thr Val Ser Trp Lys Thr Val Glu Asp Ser Ile Ile Arg Thr Gly
               20                  25                  30
   Phe Gln Gly Glu Ser Gly His Asp Ile Lys Ile Thr Ala Glu Asn Thr
           35                  40                  45
   Pro Leu Pro Ile Ala Gly Val Leu Leu Pro Thr Ile Pro Gly Lys Leu
       50                  55                  60
   Asp Val Asn Lys Ser Lys Thr His Ile Ser Thr Asn Gly Arg Lys Ile
   65                  70                  75                  80
   Arg Met Arg Cys Arg Ala Ile Asp Gly Asp Val Thr Phe Cys Arg Pro
                   85                  90                  95
   Lys Ser Pro Val Tyr Val Gly Asn Gly Gln His Ala Asn Leu His Val
               100                 105                 110
   Ala Phe Glu Arg Lys Ser Ser Glu Lys Ile His Ser Asn Glu Ile Ser
           115                 120                 125
   Ser Asp Ser Ile Ala Val Leu Gly Tyr Gln Lys Glu Val Asp His Thr
       130                 135                 140
   Lys Val Asn Ser Lys Leu Ser Leu Phe Phe Glu Ile Lys Ser
   145                 150                 155
<210> 10
   <211> 477
   <212> DNA
   <213> artificial sequence
<220>
   <223> synthetic polynucleotide (optimized coding sequence for DTR1)
<220>
   <221> CDS
   <222> (1)..(474)
<400> 10
   atg ggc aaa agc ccg ggt cat aaa acc gag ccg ttt acc cat gat ggc          48
   Met Gly Lys Ser Pro Gly His Lys Thr Glu Pro Phe Thr His Asp Gly
   1               5                   10                  15
   tat acc gtg agc tgg aac acc gtg gaa gat agc att att cgt acc ggc          96
   Tyr Thr Val Ser Trp Asn Thr Val Glu Asp Ser Ile Ile Arg Thr Gly
               20                  25                  30
   ttt cag ggc gaa agc ggc cat gat att aaa att acc gcg gaa aac acc         144
   Phe Gln Gly Glu Ser Gly His Asp Ile Lys Ile Thr Ala Glu Asn Thr
           35                  40                  45
   ccg ctg ccg att gcg ggt gtt ctg ctg ccg acc att ccg ggc aaa ctg         192
   Pro Leu Pro Ile Ala Gly Val Leu Leu Pro Thr Ile Pro Gly Lys Leu
       50                  55                  60
   gat gtg aac aaa agc aaa acc cat att agc gtg aac ggc cgt aaa att         240
   Asp Val Asn Lys Ser Lys Thr His Ile Ser Val Asn Gly Arg Lys Ile
   65                  70                  75                  80
   cgt atg cgt tgc cgt gcg att gat ggt gat gtg acc ttt tgc cgt ccg         288
   Arg Met Arg Cys Arg Ala Ile Asp Gly Asp Val Thr Phe Cys Arg Pro
                   85                  90                  95
   aaa agc ccg gtg tat gtg ggc aac ggc gtg cat gcg aac ctg cat gtg         336
   Lys Ser Pro Val Tyr Val Gly Asn Gly Val His Ala Asn Leu His Val
               100                 105                 110
   gcg ttt cat cgt agc agc agc gaa aaa atc cat agc aac gaa att agc         384
   Ala Phe His Arg Ser Ser Ser Glu Lys Ile His Ser Asn Glu Ile Ser
           115                 120                 125
   agc gat agc att ggc gtg ctg ggc tat cag aaa acc gtg gat cat acc         432
   Ser Asp Ser Ile Gly Val Leu Gly Tyr Gln Lys Thr Val Asp His Thr
       130                 135                 140
   aaa gtg aac tct aaa ctg agc ctg ttc ttc gaa atc aaa agc tga             477
   Lys Val Asn Ser Lys Leu Ser Leu Phe Phe Glu Ile Lys Ser
   145                 150                 155
<210> 11
   <211> 158
   <212> PRT
   <213> artificial sequence
<220>
   <223> Synthetic Construct
<400> 11
   Met Gly Lys Ser Pro Gly His Lys Thr Glu Pro Phe Thr His Asp Gly
   1               5                   10                  15
   Tyr Thr Val Ser Trp Asn Thr Val Glu Asp Ser Ile Ile Arg Thr Gly
               20                  25                  30
   Phe Gln Gly Glu Ser Gly His Asp Ile Lys Ile Thr Ala Glu Asn Thr
           35                  40                  45
   Pro Leu Pro Ile Ala Gly Val Leu Leu Pro Thr Ile Pro Gly Lys Leu
       50                  55                  60
   Asp Val Asn Lys Ser Lys Thr His Ile Ser Val Asn Gly Arg Lys Ile
   65                  70                  75                  80
   Arg Met Arg Cys Arg Ala Ile Asp Gly Asp Val Thr Phe Cys Arg Pro
                   85                  90                  95
   Lys Ser Pro Val Tyr Val Gly Asn Gly Val His Ala Asn Leu His Val
               100                 105                 110
   Ala Phe His Arg Ser Ser Ser Glu Lys Ile His Ser Asn Glu Ile Ser
           115                 120                 125
   Ser Asp Ser Ile Gly Val Leu Gly Tyr Gln Lys Thr Val Asp His Thr
       130                 135                 140
   Lys Val Asn Ser Lys Leu Ser Leu Phe Phe Glu Ile Lys Ser
   145                 150                 155
<210> 12
   <211> 477
   <212> DNA
   <213> artificial sequence
<220>
   <223> synthetic polynucleotide (optimized coding sequence for DTR3)
<220>
   <221> CDS
   <222> (1)..(474)
<400> 12
   atg ggc aaa agc ccg ggt cat aaa acc gag ccg tat acc cat gat ggc       48
   Met Gly Lys Ser Pro Gly His Lys Thr Glu Pro Tyr Thr His Asp Gly
   1               5                   10                  15
   tat acc gtg agc tgg aac acc gtg gaa gat agc att att cgt acc ggc       96
   Tyr Thr Val Ser Trp Asn Thr Val Glu Asp Ser Ile Ile Arg Thr Gly
               20                  25                  30
   ttt cag ggc gaa agc ggc cat gat att aaa att acc gcg gaa aac acc      144
   Phe Gln Gly Glu Ser Gly His Asp Ile Lys Ile Thr Ala Glu Asn Thr
           35                  40                  45
   ccg ctg ccg att gcg ggt gtt ctg ctg ccg acc att ccg ggc aaa ctg      192
   Pro Leu Pro Ile Ala Gly Val Leu Leu Pro Thr Ile Pro Gly Lys Leu
       50                  55                  60
   gat gtg aac aaa agc aaa acc cat att agc gtg aac ggc cgt aaa att      240
   Asp Val Asn Lys Ser Lys Thr His Ile Ser Val Asn Gly Arg Lys Ile
   65                  70                  75                  80
   cgt atg cgt tgc cgt gcg att gat ggt gat gtg acc ttt tgc cgt ccg      288
   Arg Met Arg Cys Arg Ala Ile Asp Gly Asp Val Thr Phe Cys Arg Pro
                   85                  90                  95
   aaa agc ccg gtg tat gtg ggc aac ggc gtg cat gcg aac ctg cat gtg      336
   Lys Ser Pro Val Tyr Val Gly Asn Gly Val His Ala Asn Leu His Val
               100                 105                 110
   gcg ttt cat cgt agc agc agc gaa aaa atc cat agc aac gaa att agc      384
   Ala Phe His Arg Ser Ser Ser Glu Lys Ile His Ser Asn Glu Ile Ser
           115                 120                 125
   agc gat agc att gcg gtg ctg ggc tat cag aaa acc gtg gat cat acc      432
   Ser Asp Ser Ile Ala Val Leu Gly Tyr Gln Lys Thr Val Asp His Thr
       130                 135                 140
   aaa gtg aac tct aaa ctg agc ctg ttc ttc gaa atc aaa agc tga          477
   Lys Val Asn Ser Lys Leu Ser Leu Phe Phe Glu Ile Lys Ser
   145                 150                 155
<210> 13
   <211> 158
   <212> PRT
   <213> artificial sequence
<220>
   <223> Synthetic Construct
<400> 13
   Met Gly Lys Ser Pro Gly His Lys Thr Glu Pro Tyr Thr His Asp Gly
   1               5                   10                  15
   Tyr Thr Val Ser Trp Asn Thr Val Glu Asp Ser Ile Ile Arg Thr Gly
               20                  25                  30
   Phe Gln Gly Glu Ser Gly His Asp Ile Lys Ile Thr Ala Glu Asn Thr
           35                  40                  45
   Pro Leu Pro Ile Ala Gly Val Leu Leu Pro Thr Ile Pro Gly Lys Leu
       50                  55                  60
   Asp Val Asn Lys Ser Lys Thr His Ile Ser Val Asn Gly Arg Lys Ile
   65                  70                  75                  80
   Arg Met Arg Cys Arg Ala Ile Asp Gly Asp Val Thr Phe Cys Arg Pro
                   85                  90                  95
   Lys Ser Pro Val Tyr Val Gly Asn Gly Val His Ala Asn Leu His Val
               100                 105                 110
   Ala Phe His Arg Ser Ser Ser Glu Lys Ile His Ser Asn Glu Ile Ser
           115                 120                 125
   Ser Asp Ser Ile Ala Val Leu Gly Tyr Gln Lys Thr Val Asp His Thr
       130                 135                 140
   Lys Val Asn Ser Lys Leu Ser Leu Phe Phe Glu Ile Lys Ser
   145                 150                 155
<210> 14
   <211> 477
   <212> DNA
   <213> artificial sequence
<220>
   <223> synthetic polynucleotide (optimized coding sequence for DTR8)
<220>
   <221> CDS
   <222> (1)..(474)
<400> 14
   atg ggc aaa agc ccg ggt cat aaa acc gag ccg tat acc cat gat ggc 48
   Met Gly Lys Ser Pro Gly His Lys Thr Glu Pro Tyr Thr His Asp Gly
   1 5 10 15
   tat acc gtg agc tgg aaa acc gtg gaa gat agc att att cgt acc ggc 96
   Tyr Thr Val Ser Trp Lys Thr Val Glu Asp Ser Ile Ile Arg Thr Gly
   20 25 30
   ttt cag ggc gaa agc ggc cat gat att aaa att acc gcg gaa aac acc 144
   Phe Gln Gly Glu Ser Gly His Asp Ile Lys Ile Thr Ala Glu Asn Thr
   35 40 45
   ccg ctg ccg att gcg ggt gtt ctg ctg ccg acc att ccg ggc aaa ctg 192
   Pro Leu Pro Ile Ala Gly Val Leu Leu Pro Thr Ile Pro Gly Lys Leu
   50 55 60
   gat gtg aac aaa agc aaa acc cat att agc acg aac ggc cgt aaa att 240
   Asp Val Asn Lys Ser Lys Thr His Ile Ser Thr Asn Gly Arg Lys Ile
   65 70 75 80
   cgt atg cgt tgc cgt gcg att gat ggt gat gtg acc ttt tgc cgt ccg 288
   Arg Met Arg Cys Arg Ala Ile Asp Gly Asp Val Thr Phe Cys Arg Pro
   85 90 95
   aaa agc ccg gtg tat gtg ggc aac ggc cag cat gcg aac ctg cat gtg 336
   Lys Ser Pro Val Tyr Val Gly Asn Gly Gln His Ala Asn Leu His Val
   100 105 110
   gcg ttt gaa cgt aaa agc agc gaa aaa atc cat agc aac gaa att agc 384
   Ala Phe Glu Arg Lys Ser Ser Glu Lys Ile His Ser Asn Glu Ile Ser
   115 120 125
   agc gat agc att gcg gtg ctg ggc tat cag aaa gaa gtg gat cat acc 432
   Ser Asp Ser Ile Ala Val Leu Gly Tyr Gln Lys Glu Val Asp His Thr
   130 135 140
   aaa gtg aac tct aaa ctg agc ctg ttc ttc gaa atc aaa agc tga 477
   Lys Val Asn Ser Lys Leu Ser Leu Phe Phe Glu Ile Lys Ser
   145 150 155
<210> 15
   <211> 158
   <212> PRT
   <213> artificial sequence
<220>
   <223> Synthetic Construct
<400> 15
   Met Gly Lys Ser Pro Gly His Lys Thr Glu Pro Tyr Thr His Asp Gly
   1               5                   10                  15
   Tyr Thr Val Ser Trp Lys Thr Val Glu Asp Ser Ile Ile Arg Thr Gly
               20                  25                  30
   Phe Gln Gly Glu Ser Gly His Asp Ile Lys Ile Thr Ala Glu Asn Thr
           35                  40                  45
   Pro Leu Pro Ile Ala Gly Val Leu Leu Pro Thr Ile Pro Gly Lys Leu
       50                  55                  60
   Asp Val Asn Lys Ser Lys Thr His Ile Ser Thr Asn Gly Arg Lys Ile
   65                  70                  75                  80
   Arg Met Arg Cys Arg Ala Ile Asp Gly Asp Val Thr Phe Cys Arg Pro
                   85                  90                  95
   Lys Ser Pro Val Tyr Val Gly Asn Gly Gln His Ala Asn Leu His Val
               100                 105                 110
   Ala Phe Glu Arg Lys Ser Ser Glu Lys Ile His Ser Asn Glu Ile Ser
           115                 120                 125
   Ser Asp Ser Ile Ala Val Leu Gly Tyr Gln Lys Glu Val Asp His Thr
       130                 135                 140
   Lys Val Asn Ser Lys Leu Ser Leu Phe Phe Glu Ile Lys Ser
   145                 150                 155
<210> 16
   <211> 477
   <212> DNA
   <213> artificial sequence
<220>
   <223> synthetic polynucleotide (optimized coding sequence for DTRwt)
<220>
   <221> CDS
   <222> (1)..(474)
<400> 16
   atg ggc tac agc ccg ggt cat aaa acc cag ccg ttt ctg cat gat ggc       48
   Met Gly Tyr Ser Pro Gly His Lys Thr Gln Pro Phe Leu His Asp Gly
   1               5                   10                  15
   tat gcg gtg agc tgg aac acc gtg gaa gat agc att att cgt acc ggc       96
   Tyr Ala Val Ser Trp Asn Thr Val Glu Asp Ser Ile Ile Arg Thr Gly
               20                  25                  30
   ttt cag ggc gaa agc ggc cat gat att aaa att acc gcg gaa aac acc      144
   Phe Gln Gly Glu Ser Gly His Asp Ile Lys Ile Thr Ala Glu Asn Thr
           35                  40                  45
   ccg ctg ccg att gcg ggt gtt ctg ctg ccg acc att ccg ggc aaa ctg      192
   Pro Leu Pro Ile Ala Gly Val Leu Leu Pro Thr Ile Pro Gly Lys Leu
       50                  55                  60
   gat gtg aac aaa agc aaa acc cat att agc gtg aac ggc cgt aaa att      240
   Asp Val Asn Lys Ser Lys Thr His Ile Ser Val Asn Gly Arg Lys Ile
   65                  70                  75                  80
   cgt atg cgt tgc cgt gcg att gat ggt gat gtg acc ttt tgc cgt ccg      288
   Arg Met Arg Cys Arg Ala Ile Asp Gly Asp Val Thr Phe Cys Arg Pro
                   85                  90                  95
   aaa agc ccg gtg tat gtg ggc aac ggc gtg cat gcg aac ctg cat gtg      336
   Lys Ser Pro Val Tyr Val Gly Asn Gly Val His Ala Asn Leu His Val
               100                 105                 110
   gcg ttt cat cgt agc agc agc gaa aaa atc cat agc aac gaa att agc      384
   Ala Phe His Arg Ser Ser Ser Glu Lys Ile His Ser Asn Glu Ile Ser
           115                 120                 125
   agc gat agc att ggc gtg ctg ggc tat cag aaa acc gtg gat cat acc      432
   Ser Asp Ser Ile Gly Val Leu Gly Tyr Gln Lys Thr Val Asp His Thr
       130                 135                 140
   aaa gtg aac tct aaa ctg agc ctg ttc ttc gaa atc aaa agc tga          477
   Lys Val Asn Ser Lys Leu Ser Leu Phe Phe Glu Ile Lys Ser
   145                 150                 155
<210> 17
   <211> 158
   <212> PRT
   <213> artificial sequence
<220>
   <223> Synthetic Construct
<400> 17
   Met Gly Tyr Ser Pro Gly His Lys Thr Gln Pro Phe Leu His Asp Gly
   1               5                   10                  15
   Tyr Ala Val Ser Trp Asn Thr Val Glu Asp Ser Ile Ile Arg Thr Gly
               20                  25                  30
   Phe Gln Gly Glu Ser Gly His Asp Ile Lys Ile Thr Ala Glu Asn Thr
           35                  40                  45
   Pro Leu Pro Ile Ala Gly Val Leu Leu Pro Thr Ile Pro Gly Lys Leu
       50                  55                  60
   Asp Val Asn Lys Ser Lys Thr His Ile Ser Val Asn Gly Arg Lys Ile
   65                  70                  75                  80
   Arg Met Arg Cys Arg Ala Ile Asp Gly Asp Val Thr Phe Cys Arg Pro
                   85                  90                  95
   Lys Ser Pro Val Tyr Val Gly Asn Gly Val His Ala Asn Leu His Val
               100                 105                 110
   Ala Phe His Arg Ser Ser Ser Glu Lys Ile His Ser Asn Glu Ile Ser
           115                 120                 125
   Ser Asp Ser Ile Gly Val Leu Gly Tyr Gln Lys Thr Val Asp His Thr
       130                 135                 140
   Lys Val Asn Ser Lys Leu Ser Leu Phe Phe Glu Ile Lys Ser
   145                 150                 155
<210> 18
   <211> 15
   <212> PRT
   <213> artificial sequence
<220>
   <223> synthetic peptide (peptide 1: DTR 378-392)
<400> 18
   Met Gly Tyr Ser Pro Gly His Lys Thr Gln Pro Phe Leu His Asp
   1               5                   10                  15
<210> 19
   <211> 15
   <212> PRT
   <213> artificial sequence
<220>
   <223> synthetic peptide (peptide 2: DTR 385-399)
<400> 19
   Lys Thr Gln Pro Phe Leu His Asp Gly Tyr Ala Val Ser Trp Asn
   1               5                   10                  15
<210> 20
   <211> 15
   <212> PRT
   <213> artificial sequence
<220>
   <223> synthetic peptide (peptide 3: DTR 391-405)
<400> 20
   His Asp Gly Tyr Ala Val Ser Trp Asn Thr Val Glu Asp Ser Ile
   1               5                   10                  15
<210> 21
   <211> 15
   <212> PRT
   <213> artificial sequence
<220>
   <223> synthetic peptide (peptide 4: DTR 397-411)
<400> 21
   Ser Trp Asn Thr Val Glu Asp Ser Ile Ile Arg Thr Gly Phe Gln
   1               5                   10                  15
<210> 22
   <211> 15
   <212> PRT
   <213> artificial sequence
<220>
   <223> synthetic peptide (peptide 5: DTR 403-417)
<400> 22
   Asp Ser Ile Ile Arg Thr Gly Phe Gln Gly Glu Ser Gly His Asp
   1               5                   10                  15
<210> 23
   <211> 15
   <212> PRT
   <213> artificial sequence
<220>
   <223> synthetic peptide (peptide 6: DTR 409-423)
<400> 23
   Gly Phe Gln Gly Glu Ser Gly His Asp Ile Lys Ile Thr Ala Glu
   1               5                   10                  15
<210> 24
   <211> 15
   <212> PRT
   <213> artificial sequence
<220>
   <223> synthetic peptide (peptide 7: DTR 415-429)
<400> 24
   Gly His Asp Ile Lys Ile Thr Ala Glu Asn Thr Pro Leu Pro Ile
   1               5                   10                  15
<210> 25
   <211> 15
   <212> PRT
   <213> artificial sequence
<220>
   <223> synthetic peptide (peptide 8: DTR 421-435)
<400> 25
   Thr Ala Glu Asn Thr Pro Leu Pro Ile Ala Gly Val Leu Leu Pro
   1               5                   10                  15
<210> 26
   <211> 15
   <212> PRT
   <213> artificial sequence
<220>
   <223> synthetic peptide (peptide 9: DTR 427-441)
<400> 26
   Leu Pro Ile Ala Gly Val Leu Leu Pro Thr Ile Pro Gly Lys Leu
   1               5                   10                  15
<210> 27
   <211> 15
   <212> PRT
   <213> artificial sequence
<220>
   <223> synthetic peptide (peptide 10: DTR 433-447)
<400> 27
   Leu Leu Pro Thr Ile Pro Gly Lys Leu Asp Val Asn Lys Ser Lys
   1               5                   10                  15
<210> 28
   <211> 15
   <212> PRT
   <213> artificial sequence
<220>
   <223> synthetic peptide (peptide 11: DTR 439-453)
<400> 28
   Gly Lys Leu Asp Val Asn Lys Ser Lys Thr His Ile Ser Val Asn
   1 5 10 15
<210> 29
   <211> 15
   <212> PRT
   <213> artificial sequence
<220>
   <223> synthetic peptide (peptide 12: DTR 445-459)
<400> 29
   Lys Ser Lys Thr His Ile Ser Val Asn Gly Arg Lys Ile Arg Met
   1 5 10 15
<210> 30
   <211> 15
   <212> PRT
   <213> artificial sequence
<220>
   <223> synthetic peptide (peptide 13: DTR 451-465)
<400> 30
   Ser Val Asn Gly Arg Lys Ile Arg Met Arg Cys Arg Ala Ile Asp
   1 5 10 15
<210> 31
   <211> 15
   <212> PRT
   <213> artificial sequence
<220>
   <223> synthetic peptide (peptide 14: DTR 457-471)
<400> 31
   Ile Arg Met Arg Cys Arg Ala Ile Asp Gly Asp Val Thr Phe Cys
   1 5 10 15
<210> 32
   <211> 15
   <212> PRT
   <213> artificial sequence
<220>
   <223> synthetic peptide (peptide 15: DTR 463-477)
<400> 32
   Ala Ile Asp Gly Asp Val Thr Phe Cys Arg Pro Lys Ser Pro Val
   1 5 10 15
<210> 33
   <211> 15
   <212> PRT
   <213> artificial sequence
<220>
   <223> synthetic peptide (peptide 16: DTR 469-483)
<400> 33
   Thr Phe Cys Arg Pro Lys Ser Pro Val Tyr Val Gly Asn Gly Val
   1               5                   10                  15
<210> 34
   <211> 15
   <212> PRT
   <213> artificial sequence
<220>
   <223> synthetic peptide (peptide 17: DTR 475-489)
<400> 34
   Ser Pro Val Tyr Val Gly Asn Gly Val His Ala Asn Leu His Val
   1               5                   10                  15
<210> 35
   <211> 15
   <212> PRT
   <213> artificial sequence
<220>
   <223> synthetic peptide (peptide 18: DTR 482-496)
<400> 35
   Gly Val His Ala Asn Leu His Val Ala Phe His Arg Ser Ser Ser
   1               5                   10                  15
<210> 36
   <211> 15
   <212> PRT
   <213> artificial sequence
<220>
   <223> synthetic peptide (peptide 19: DTR 488-502)
<400> 36
   His Val Ala Phe His Arg Ser Ser Ser Glu Lys Ile His Ser Asn
   1               5                   10                  15
<210> 37
   <211> 15
   <212> PRT
   <213> artificial sequence
<220>
   <223> synthetic peptide (peptide 20: DTR 494-508)
<400> 37
   Ser Ser Ser Glu Lys Ile His Ser Asn Glu Ile Ser Ser Asp Ser
   1               5                   10                  15
<210> 38
   <211> 15
   <212> PRT
   <213> artificial sequence
<220>
   <223> synthetic peptide (peptide 21: DTR 500-514)
<400> 38
   His Ser Asn Glu Ile Ser Ser Asp Ser Ile Gly Val Leu Gly Tyr
   1               5                   10                  15
<210> 39
   <211> 15
   <212> PRT
   <213> artificial sequence
<220>
   <223> synthetic peptide (peptide 22: DTR 506-520)
<400> 39
   Ser Asp Ser Ile Gly Val Leu Gly Tyr Gln Lys Thr Val Asp His
   1               5                   10                  15
<210> 40
   <211> 15
   <212> PRT
   <213> artificial sequence
<220>
   <223> synthetic peptide (peptide 23: DTR 512-526)
<400> 40
   Leu Gly Tyr Gln Lys Thr Val Asp His Thr Lys Val Asn Ser Lys
   1               5                   10                  15
<210> 41
   <211> 15
   <212> PRT
   <213> artificial sequence
<220>
   <223> synthetic peptide (peptide 24: DTR 518-532)
<400> 41
   Val Asp His Thr Lys Val Asn Ser Lys Leu Ser Leu Phe Phe Glu
   1               5                   10                  15
<210> 42
   <211> 15
   <212> PRT
   <213> artificial sequence
<220>
   <223> synthetic peptide (peptide 25: DTR 521-535)
<400> 42
   Thr Lys Val Asn Ser Lys Leu Ser Leu Phe Phe Glu Ile Lys Ser
   1               5                   10                  15
<210> 43
   <211> 26
   <212> PRT
   <213> artificial sequence
<220>
   <223> synthetic peptide (DTRwt 378-403)
<400> 43
   Met Gly Tyr Ser Pro Gly His Lys Thr Gln Pro Phe Leu His Asp Gly
   1               5                   10                  15
   Tyr Ala Val Ser Trp Asn Thr Val Glu Asp
               20                  25
<210> 44
   <211> 26
   <212> PRT
   <213> artificial sequence
<220>
   <223> synthetic peptide (DTR1 378-403)
<400> 44
   Met Gly Lys Ser Pro Gly His Lys Thr Glu Pro Phe Thr His Asp Gly
   1               5                   10                  15
   Tyr Thr Val Ser Trp Asn Thr Val Glu Asp
               20                  25
<210> 45
   <211> 9
   <212> PRT
   <213> artificial sequence
<220>
   <223> synthetic peptide (DTRwt 389-397)
<400> 45
   Phe Leu His Asp Gly Tyr Ala Val Ser
   1               5
<210> 46
   <211> 9
   <212> PRT
   <213> artificial sequence
<220>
   <223> synthetic peptide (DTRwt 394-402)
<400> 46
   Tyr Ala Val Ser Trp Asn Thr Val Glu
   1               5
<210> 47
   <211> 9
   <212> PRT
   <213> artificial sequence
<220>
   <223> synthetic peptide (DTR1 389-397)
<400> 47
   Phe Thr His Asp Gly Tyr Thr Val Ser
   1               5
<210> 48
   <211> 9
   <212> PRT
   <213> artificial sequence
<220>
   <223> synthetic peptide (DTR1 394-402)
<400> 48
   Tyr Thr Val Ser Trp Asn Thr Val Glu
   1               5
<210> 49
   <211> 21
   <212> PRT
   <213> artificial sequence
<220>
   <223> synthetic peptide (DTR1 391-411)
<400> 49
   His Asp Gly Tyr Thr Val Ser Trp Asn Thr Val Glu Asp Ser Ile Ile
   1               5                   10                  15
   Arg Thr Gly Phe Gln
               20
<210> 50
   <211> 21
   <212> PRT
   <213> artificial sequence
<220>
   <223> synthetic peptide (N399K variant 391-411)
<400> 50
   His Asp Gly Tyr Thr Val Ser Trp Lys Thr Val Glu Asp Ser Ile Ile
   1               5                   10                  15
   Arg Thr Gly Phe Gln
               20
<210> 51
   <211> 21
   <212> PRT
   <213> artificial sequence
<220>
   <223> synthetic peptide (V401Q variant 391-411)
<400> 51
   His Asp Gly Tyr Thr Val Ser Trp Asn Thr Gln Glu Asp Ser Ile Ile
   1               5                   10                  15
   Arg Thr Gly Phe Gln
               20
<210> 52
   <211> 21
   <212> PRT
   <213> artificial sequence
<220>
   <223> synthetic peptide (N399K/V401Q variant 391-411)
<400> 52
   His Asp Gly Tyr Thr Val Ser Trp Lys Thr Gln Glu Asp Ser Ile Ile
   1               5                   10                  15
   Arg Thr Gly Phe Gln
               20
<210> 53
   <211> 9
   <212> PRT
   <213> artificial sequence
<220>
   <223> synthetic peptide (DTR1 396-404)
<400> 53
   Val Ser Trp Asn Thr Val Glu Asp Ser
   1 5
<210> 54
   <211> 9
   <212> PRT
   <213> artificial sequence
<220>
   <223> synthetic peptide (DTR1 394-402)
<400> 54
   Tyr Thr Val Ser Trp Asn Thr Val Glu
   1 5
<210> 55
   <211> 9
   <212> PRT
   <213> artificial sequence
<220>
   <223> synthetic peptide (DTR1 398-406)
<400> 55
   Trp Asn Thr Val Glu Asp Ser Ile Ile
   1 5
<210> 56
   <211> 9
   <212> PRT
   <213> artificial sequence
<220>
   <223> synthetic peptide (N399K variant 398-406)
<400> 56
   Trp Lys Thr Val Glu Asp Ser Ile Ile
   1 5
<210> 57
   <211> 9
   <212> PRT
   <213> artificial sequence
<220>
   <223> synthetic peptide (N399K variant 396-404)
<400> 57
   Val Ser Trp Lys Thr Val Glu Asp Ser
   1 5
<210> 58
   <211> 9
   <212> PRT
   <213> artificial sequence
<220>
   <223> synthetic peptide (V401Q variant 394-402)
<400> 58
   Tyr Thr Val Ser Trp Asn Thr Gln Glu
   1 5
<210> 59
   <211> 15
   <212> PRT
   <213> artificial sequence
<220>
   <223> synthetic peptide (DTR1 427-441)
<400> 59
   Leu Pro Ile Ala Gly Val Leu Leu Pro Thr Ile Pro Gly Lys Leu
   1 5 10 15
<210> 60
   <211> 15
   <212> PRT
   <213> artificial sequence
<220>
   <223> synthetic peptide (L427Q variant 427-441)
<400> 60
   Gln Pro Ile Ala Gly Val Leu Leu Pro Thr Ile Pro Gly Lys Leu
   1 5 10 15
<210> 61
   <211> 15
   <212> PRT
   <213> artificial sequence
<220>
   <223> synthetic peptide (T436K variant 427-441)
<400> 61
   Leu Pro Ile Ala Gly Val Leu Leu Pro Lys Ile Pro Gly Lys Leu
   1 5 10 15
<210> 62
   <211> 15
   <212> PRT
   <213> artificial sequence
<220>
   <223> synthetic peptide (L427Q/T436K variant 427-441)
<400> 62
   Gln Pro Ile Ala Gly Val Leu Leu Pro Lys Ile Pro Gly Lys Leu
   1 5 10 15
<210> 63
   <211> 9
   <212> PRT
   <213> artificial sequence
<220>
   <223> synthetic peptide (DTR1 432-440)
<400> 63
   Val Leu Leu Pro Thr Ile Pro Gly Lys
   1 5
<210> 64
   <211> 9
   <212> PRT
   <213> artificial sequence
<220>
   <223> synthetic peptide (T436K variant 432-440)
<400> 64
   Val Leu Leu Pro Lys Ile Pro Gly Lys
   1 5
<210> 65
   <211> 9
   <212> PRT
   <213> artificial sequence
<220>
   <223> synthetic peptide (DTR1 433-441)
<400> 65
   Leu Leu Pro Thr Ile Pro Gly Lys Leu
   1 5
<210> 66
   <211> 9
   <212> PRT
   <213> artificial sequence
<220>
   <223> synthetic peptide (DTR1 427-435)
<400> 66
   Leu Pro Ile Ala Gly Val Leu Leu Pro
   1 5
<210> 67
   <211> 15
   <212> PRT
   <213> artificial sequence
<220>
   <223> synthetic peptide (DTR1 451-465)
<400> 67
   Ser Val Asn Gly Arg Lys Ile Arg Met Arg Cys Arg Ala Ile Asp
   1               5                   10                  15
<210> 68
   <211> 15
   <212> PRT
   <213> artificial sequence
<220>
   <223> synthetic peptide (I457D variant 451-465)
<400> 68
   Ser Val Asn Gly Arg Lys Asp Arg Met Arg Cys Arg Ala Ile Asp
   1               5                   10                  15
<210> 69
   <211> 15
   <212> PRT
   <213> artificial sequence
<220>
   <223> synthetic peptide (I457E variant 451-465)
<400> 69
   Ser Val Asn Gly Arg Lys Glu Arg Met Arg Cys Arg Ala Ile Asp
   1               5                   10                  15
<210> 70
   <211> 15
   <212> PRT
   <213> artificial sequence
<220>
   <223> synthetic peptide (V452T variant 451-465)
<400> 70
   Ser Thr Asn Gly Arg Lys Ile Arg Met Arg Cys Arg Ala Ile Asp
   1               5                   10                  15
<210> 71
   <211> 15
   <212> PRT
   <213> artificial sequence
<220>
   <223> synthetic peptide (R460T variant 451-465)
<400> 71
   Ser Val Asn Gly Arg Lys Ile Arg Met Thr Cys Arg Ala Ile Asp
   1 5 10 15
<210> 72
   <211> 15
   <212> PRT
   <213> artificial sequence
<220>
   <223> synthetic peptide (A463D variant 451-465)
<400> 72
   Ser Val Asn Gly Arg Lys Ile Arg Met Arg Cys Arg Asp Ile Asp
   1 5 10 15
<210> 73
   <211> 15
   <212> PRT
   <213> artificial sequence
<220>
   <223> synthetic peptide (V452T/R460T/A463D variant 451-465)
<400> 73
   Ser Thr Asn Gly Arg Lys Ile Arg Met Thr Cys Arg Asp Ile Asp
   1 5 10 15
<210> 74
   <211> 9
   <212> PRT
   <213> artificial sequence
<220>
   <223> synthetic peptide (DTR1 457-465)
<400> 74
   Ile Arg Met Arg Cys Arg Ala Ile Asp
   1 5
<210> 75
   <211> 9
   <212> PRT
   <213> artificial sequence
<220>
   <223> synthetic peptide (R460T variant 457-465)
<400> 75
   Ile Arg Met Thr Cys Arg Ala Ile Asp
   1 5
<210> 76
   <211> 9
   <212> PRT
   <213> artificial sequence
<220>
   <223> synthetic peptide (A463D variant 457-465)
<400> 76
   Ile Arg Met Arg Cys Arg Asp Ile Asp
   1 5
<210> 77
   <211> 9
   <212> PRT
   <213> artificial sequence
<220>
   <223> synthetic peptide (V452T/R460T/A463D variant 451-465)
<400> 77
   Ile Arg Met Thr Cys Arg Asp Ile Asp
   1 5
<210> 78
   <211> 9
   <212> PRT
   <213> artificial sequence
<220>
   <223> synthetic peptide (DTR1 452-460)
<400> 78
   Val Asn Gly Arg Lys Ile Arg Met Arg
   1 5
<210> 79
   <211> 9
   <212> PRT
   <213> artificial sequence
<220>
   <223> synthetic peptide (R460T variant 452-460)
<400> 79
   Val Asn Gly Arg Lys Ile Arg Met Thr
   1 5
<210> 80
   <211> 28
   <212> PRT
   <213> artificial sequence
<220>
   <223> synthetic peptide (DTR1 475-502)
<400> 80
   Ser Pro Val Tyr Val Gly Asn Gly Val His Ala Asn Leu His Val Ala
   1               5                   10                  15
   Phe His Arg Ser Ser Ser Glu Lys Ile His Ser Asn
               20                  25
<210> 81
   <211> 28
   <212> PRT
   <213> artificial sequence
<220>
   <223> synthetic peptide (Y478T variant 475-502)
<400> 81
   Ser Pro Val Thr Val Gly Asn Gly Val His Ala Asn Leu His Val Ala
   1               5                   10                  15
   Phe His Arg Ser Ser Ser Glu Lys Ile His Ser Asn
               20                  25
<210> 82
   <211> 28
   <212> PRT
   <213> artificial sequence
<220>
   <223> synthetic peptide (V483D variant 475-502)
<400> 82
   Ser Pro Val Tyr Val Gly Asn Gly Asp His Ala Asn Leu His Val Ala
   1               5                   10                  15
   Phe His Arg Ser Ser Ser Glu Lys Ile His Ser Asn
               20                  25
<210> 83
   <211> 28
   <212> PRT
   <213> artificial sequence
<220>
   <223> synthetic peptide (V483E variant 475-502)
<400> 83
   Ser Pro Val Tyr Val Gly Asn Gly Glu His Ala Asn Leu His Val Ala
   1               5                   10                  15
   Phe His Arg Ser Ser Ser Glu Lys Ile His Ser Asn
               20                  25
<210> 84
   <211> 28
   <212> PRT
   <213> artificial sequence
<220>
   <223> synthetic peptide (V483H variant 475-502)
<400> 84
   Ser Pro Val Tyr Val Gly Asn Gly His His Ala Asn Leu His Val Ala
   1               5                   10                  15
   Phe His Arg Ser Ser Ser Glu Lys Ile His Ser Asn
               20                  25
<210> 85
   <211> 28
   <212> PRT
   <213> artificial sequence
<220>
   <223> synthetic peptide (V483Q variant 475-502)
<400> 85
   Ser Pro Val Tyr Val Gly Asn Gly Gln His Ala Asn Leu His Val Ala
   1               5                   10                  15
   Phe His Arg Ser Ser Ser Glu Lys Ile His Ser Asn
               20                  25
<210> 86
   <211> 28
   <212> PRT
   <213> artificial sequence
<220>
   <223> synthetic peptide (A490G variant 475-502)
<400> 86
   Ser Pro Val Tyr Val Gly Asn Gly Val His Ala Asn Leu His Val Gly
   1               5                   10                  15
   Phe His Arg Ser Ser Ser Glu Lys Ile His Ser Asn
               20                  25
<210> 87
   <211> 28
   <212> PRT
   <213> artificial sequence
<220>
   <223> synthetic peptide (H492E variant 475-502)
<400> 87
   Ser Pro Val Tyr Val Gly Asn Gly Val His Ala Asn Leu His Val Ala
   1               5                   10                  15
   Phe Glu Arg Ser Ser Ser Glu Lys Ile His Ser Asn
               20                  25
<210> 88
   <211> 28
   <212> PRT
   <213> artificial sequence
<220>
   <223> synthetic peptide (S494K variant 475-502)
<400> 88
   Ser Pro Val Tyr Val Gly Asn Gly Val His Ala Asn Leu His Val Ala
   1               5                   10                  15
   Phe His Arg Lys Ser Ser Glu Lys Ile His Ser Asn
               20                  25
<210> 89
   <211> 28
   <212> PRT
   <213> artificial sequence
<220>
   <223> synthetic peptide (S496K variant 475-502)
<400> 89
   Ser Pro Val Tyr Val Gly Asn Gly Val His Ala Asn Leu His Val Ala
   1               5                   10                  15
   Phe His Arg Ser Ser Lys Glu Lys Ile His Ser Asn
               20                  25
<210> 90
   <211> 28
   <212> PRT
   <213> artificial sequence
<220>
   <223> synthetic peptide (Y478T/A490G/H492E/S494K/S496K variant 475-502)
<400> 90
   Ser Pro Val Thr Val Gly Asn Gly Val His Ala Asn Leu His Val Gly
   1               5                   10                  15
   Phe Glu Arg Lys Ser Lys Glu Lys Ile His Ser Asn
               20                  25
<210> 91
   <211> 9
   <212> PRT
   <213> artificial sequence
<220>
   <223> synthetic peptide (DTR1 478-486)
<400> 91
   Tyr Val Gly Asn Gly Val His Ala Asn
   1               5
<210> 92
   <211> 9
   <212> PRT
   <213> artificial sequence
<220>
   <223> synthetic peptide (DTR1 491-499)
<400> 92
   Phe His Arg Ser Ser Ser Glu Lys Ile
   1               5
<210> 93
   <211> 9
   <212> PRT
   <213> artificial sequence
<220>
   <223> synthetic peptide (DTR1 477-485)
<400> 93
   Val Tyr Val Gly Asn Gly Val His Ala
   1               5
<210> 94
   <211> 9
   <212> PRT
   <213> artificial sequence
<220>
   <223> synthetic peptide (DTR1 487-495)
<400> 94
   Leu His Val Ala Phe His Arg Ser Ser
   1               5
<210> 95
   <211> 9
   <212> PRT
   <213> artificial sequence
<220>
   <223> synthetic peptide (V483D variant 478-486)
<400> 95
   Tyr Val Gly Asn Gly Asp His Ala Asn
   1               5
<210> 96
   <211> 9
   <212> PRT
   <213> artificial sequence
<220>
   <223> synthetic peptide (V483H variant 477-485)
<400> 96
   Val Tyr Val Gly Asn Gly His His Ala
   1               5
<210> 97
   <211> 9
   <212> PRT
   <213> artificial sequence
<220>
   <223> synthetic peptide (V483Q variant 477-485)
<400> 97
   Val Tyr Val Gly Asn Gly Gln His Ala
   1               5
<210> 98
   <211> 9
   <212> PRT
   <213> artificial sequence
<220>
   <223> synthetic peptide (V483Q variant 478-486)
<400> 98
   Tyr Val Gly Asn Gly Gln His Ala Asn
   1               5
<210> 99
   <211> 9
   <212> PRT
   <213> artificial sequence
<220>
   <223> synthetic peptide (A490G variant 487-495)
<400> 99
   Leu His Val Gly Phe His Arg Ser Ser
   1               5
<210> 100
   <211> 8
   <212> PRT
   <213> artificial sequence
<220>
   <223> synthetic peptide (H492E variant 491-499)
<400> 100
   Phe Glu Arg Ser Ser Ser Glu Lys
   1               5
<210> 101
   <211> 9
   <212> PRT
   <213> artificial sequence
<220>
   <223> synthetic peptide (H492E variant 487-495)
<400> 101
   Leu His Val Ala Phe Glu Arg Ser Ser
   1               5
<210> 102
   <211> 9
   <212> PRT
   <213> artificial sequence
<220>
   <223> synthetic peptide (S494K variant 491-499)
<400> 102
   Phe His Arg Lys Ser Ser Glu Lys Ile
   1               5
<210> 103
   <211> 9
   <212> PRT
   <213> artificial sequence
<220>
   <223> synthetic peptide (S494K variant 487-495)
<400> 103
   Leu His Val Ala Phe His Arg Lys Ser
   1               5
<210> 104
   <211> 9
   <212> PRT
   <213> artificial sequence
<220>
   <223> synthetic peptide (S494K variant 489-497)
<400> 104
   Val Ala Phe His Arg Lys Ser Ser Glu
   1 5
<210> 105
   <211> 9
   <212> PRT
   <213> artificial sequence
<220>
   <223> synthetic peptide (S496K variant 491-499)
<400> 105
   Phe His Arg Ser Ser Lys Glu Lys Ile
   1 5
<210> 106
   <211> 15
   <212> PRT
   <213> artificial sequence
<220>
   <223> synthetic peptide (DTR1 506-520)
<400> 106
   Ser Asp Ser Ile Gly Val Leu Gly Tyr Gln Lys Thr Val Asp His
   1 5 10 15
<210> 107
   <211> 15
   <212> PRT
   <213> artificial sequence
<220>
   <223> synthetic peptide (T517D variant 506-520)
<400> 107
   Ser Asp Ser Ile Gly Val Leu Gly Tyr Gln Lys Asp Val Asp His
   1 5 10 15
<210> 108
   <211> 15
   <212> PRT
   <213> artificial sequence
<220>
   <223> synthetic peptide (T517E variant 506-520)
<400> 108
   Ser Asp Ser Ile Gly Val Leu Gly Tyr Gln Lys Glu Val Asp His
   1 5 10 15
<210> 109
   <211> 9
   <212> PRT
   <213> artificial sequence
<220>
   <223> synthetic peptide (DTR1 512-520)
<400> 109
   Leu Gly Tyr Gln Lys Thr Val Asp His
   1               5

## Description

La présente invention est relative à une protéine recombinante ligand inhibiteur de l'HB-EGF (Heparin-Binding Epidermal Growth Factor like), issue du domaine R de la toxine diphtérique, utile pour le traitement et le diagnostic des maladies impliquant l'activation de la voie HB-EGF/EGFR.

L'HB-EGF est un facteur de croissance exprimé à la surface des cellules de l'organisme sous la forme d'une protéine membranaire précurseur, le pro-HB-EGF qui est le récepteur naturel de la toxine diphtérique.

La toxine diphtérique (DT) est une exotoxine de 535 acides aminés (SEQ ID NO : 1) composée d'un fragment A (N-terminal) et d'un fragment B (C-terminal). Le fragment A comprend le domaine catalytique (C ou DTA/DT-A ; résidus 1 à 193) et le fragment B comprend le domaine de translocation (T ; N-terminal ; résidus 202 à 378), et le domaine de liaison au récepteur cellulaire (R ou DTR ; C-terminal ; résidus 379-386 à 535). DT se fixe à la surface des cellules par liaison de son domaine R au pro-HB-EGF ; après activation de la DT par clivage protéolytique entre les fragments A et B, le domaine T permet la translocation du fragment A dans le cytoplasme. Une fois dans le cytoplasme, le domaine catalytique porté par le fragment A bloque la synthèse protéique en inactivant le facteur d'élongation EF2, provoquant ainsi la mort des cellules. L'étude de mutants naturels ou synthétiques de DT a montré que la liaison de DT au récepteur pro-HB-EGF est abolie par la substitution du résidu S508 (S508F) ou de résidus de la boucle de la région K516-F530 de DTR (K516A, K516E, F530A, F530S et S525F) mais pas par la délétion des 4 résidus C-terminaux (résidus E532-S535; Shen et al., J. Biol. Chem., 1994, 269, 29077-29084). En outre, le mutant naturel de DT comprenant la mutation G52E, dénommé CRM197, a une activité catalytique fortement diminuée (Giannini et al., N.A.R., 1984, 12, 4063-4069).

Lors de processus inflammatoires impliquant l'HB-EGF, la protéine membranaire pro-HB-EGF est clivée par une protéase et l'HB-EGF est libéré de la surface cellulaire, sous la forme d'une protéine sécrétée. Il se lie alors de manière autocrine ou paracrine sur les sous-types ErbB1 (HER1 ou EGFR) et ErbB4 (HER4) de la famille des récepteurs à l'EGF (famille EGFR ou EGFR), exprimés dans pratiquement tous les tissus de l'organisme. Il existe au moins dix ligands de l'EGFR en plus de l'HB-EGF, à savoir l'EGF, le TGF-alpha, l'amphiréguline, la bétacelluline, l'epigen, l'épiréguline et les neurégulines -1, -2, -3 et -4. C'est le contexte local qui détermine sa stimulation par l'un ou l'autre de ces ligands.

L'activation de la voie HB-EGF/EGFR qui est associée à l'augmentation de l'expression du pro-HB-EGF et/ou la libération de l'HB-EGF, entraine une prolifération cellulaire délétère responsable de nombreuses pathologies :
- les glomérulonéphrites rapidement progressives (GNRP) au cours desquelles la prolifération des cellules rénales glomérulaires (podocytes) aboutit à la destruction du rein (Feng et al., J. Clin. Invest., 2000, 105, 341-350; Bollee et al., Nat. Med., 2011, 17, 1242-1250),
- des cancers, incluant le cancer de l'ovaire, de l'endomètre, du sein, de l'utérus (adénocarcinome utérin), de la vessie, de l'estomac, de la peau (mélanome malin), du cerveau (glioblastome) et du poumon (Yotsumoto et al., Biochem Biophys Res Commun, 2008, 365, 555-561; Miyamoto et al., Adv. Exp. Med. Biol., 2008, 622, 281-295 ; Miyamoto et al., Anticancer Res., 2009, 29, 823-830 ; Fu et al., Proc. Natl. Acad. Sci. U S A, 1999, 96, 5716-5721; Tsujioka et al., Anticancer. Res., 2010, 30, 3107-3112),
- le vasospasme associé aux contusions cérébrales (Chansel et al., FASEB J., 2006, 20, 1936-1938),
- l'hypertrophie cardiaque (Asakura et al., Nat. Med., 2002, 8, 35-40 ; Hamaoka et al., J. Biochem., 2010, 148, 55-69),
- l'hyperplasie des cellules musculaires lisses (Miyagawa et al., J. Clin. Invest., 1995, 95, 404-411; Hamaoka et al., J. Biochem., 2010, 148, 55-69),
- l'hypertension pulmonaire (Powell et al., Am. J. Pathol., 1993, 143, 784-793; Hamaoka et al., J. Biochem., 2010, 148, 55-69),
- les rétinopathies diabétiques, et
- la resténose artérielle.

Du fait de son implication dans un nombre croissant de maladies, l'HB-EGF représente une cible thérapeutique. Il existe actuellement deux types de molécules disponibles pour bloquer l'action de l'HB-EGF sur l'EGFR, des inhibiteurs de l'EGFR et des inhibiteurs de l'HB-EGF. Ces molécules potentiellement utilisables pour traiter les pathologies impliquant une activation de l'EGFR par l'HB-EGF (voie HB-EGF/EGFR) présentent toutefois un certain nombre d'inconvénients majeurs.

### Inhibiteurs de l'EGFR

Deux types d'inhibiteurs de l'EGFR pourraient être théoriquement utilisés pour bloquer la stimulation de l'EGFR par l'HB-EGF : les inhibiteurs de tyrosines kinases réversibles agissant sur l'EGFR, comme l'erlotinib, et les anticorps dirigés contre l'EGFR, comme le cetuximab (Ciardiello and Tortora, N. Engl. J. Med., 358, 1160-1174; WO 2008/053270).

Toutefois, ces molécules qui agissent sur la tyrosine kinase de l'EGFR (petites molécules) ou sur l'EGFR lui-même (anticorps monoclonaux) ne sont pas spécifiques de la voie HB-EGF/EGFR, étant donné qu'elles bloquent l'activation de l'EGFR quelle que soit l'origine de sa stimulation, indépendamment du ligand l'activant et quel que soit ce ligand. Du fait de leur manque de spécificité, les inhibiteurs de l'EGFR produisent des effets secondaires significatifs. A titre d'exemple, on peut citer les effets secondaires suivants rapportés, respectivement pour l'erlotinib et le cetuximab : neutropénie, thrombocytopénie, anémie, oedèmes, nausées, vomissements, maux de tête, prurit et douleurs musculo-squelettiques ; fièvre, frissons, urticaire, prurits, éruption cutanée, hypotension, bronchospasme, dyspnée, oedèmes, confusion, choc anaphylactique et arrêt cardiaque.

De plus, les anticorps ont une taille trop importante (150 kDa) pour atteindre leur cible dans le cas des GNRP étant donné que l'EGFR stimulé par l'HB-EGF est exprimé par les podocytes dans les glomérules du rein et que le seuil de filtration glomérulaire est d'environ 68 kDa. De la même manière, la pénétration des anticorps dans des tumeurs solides est limitée du fait de leur taille.

### Inhibiteurs de l'HB-EGF

Chez la souris, l'invalidation du gène du pro-HB-EGF empêche le déclenchement d'une GNRP provoquée (Bollee et al., Nat. Med., 2011, 17, 1242-1250), justifiant ainsi l'intérêt de développer un ligand inhibiteur de l'HB-EGF et du pro-HB-EGF pour le traitement des pathologies impliquant l'activation de la voie HB-EGF/EGFR.

Il existe actuellement deux types d'inhibiteurs de HB-EGF, potentiellement utilisables pour bloquer la stimulation de l'EGFR par l'HB-EGF : les anticorps dirigés contre l'HB-EGF (WO 2009/040134; WO 2011/21381; WO 2009/72628 ; EP 2221374 ; EP 2281001 ; EP 2093237 ; EP2078731 ; EP 2039704 ; WO 2008/053270) et CRM197 (US 7,700,546 ; US 2006/0270600 ; EP 1894575).

Les anticorps dirigés contre l'HB-EGF sont plus spécifiques que les inhibiteurs de l'EGFR étant donné qu'ils bloquent uniquement l'activation de la voie HB-EGF/EGFR mais ils présentent les mêmes inconvénients que les anticorps anti-EGFR, liés à leur taille trop importante.

CRM197 est un ligand naturel de l'HB-EGF capable de bloquer sa liaison à l'EGFR. Elle est actuellement utilisée dans des essais cliniques pour bloquer l'HB-EGF afin de lutter contre le cancer de l'ovaire (Koshikawa et al., Cancer Sci., 2011, 102, 111-116; Tsujioka et al., Anticancer Res., 2011, 31, 2461-2465).

Toutefois, CRM197 présente des inconvénients de toxicité résiduelle, de taille, d'affinité, d'immunogénicité et d'antigénicité.

La toxicité résiduelle de CRM197 est 10⁶ fois plus faible que celle de la toxine diphtérique sauvage (Kageyama et al., J. Biochem., 2007, 142, 95-104). Ceci est cependant non négligeable puisque la toxine sauvage est extrêmement puissante avec une dose létale 50 (DL50) de 5pM en culture de cellules de primate. CRM197 est donc toxique à des doses micromolaires (µM). Ceci peut présenter un risque si elle est administrée chez l'homme à dose élevée (Kageyama et al., J. Biochem., 2007, 142, 95-104).

CRM197 a une masse moléculaire de 58 kDa, passant difficilement le seuil de la filtration glomérulaire. Elle ne peut donc pas être utilisée pour traiter les GNRP ou d'autres pathologies comme les tumeurs solides, où la pénétration tissulaire est un facteur important pour l'efficacité thérapeutique.

CRM197 possède une affinité moyenne pour HB-EGF (Kd = 27 nM ; Brooke et al., Biochem. Biophys. Res. Commun., 1998, 248, 297-302). Cette affinité est inférieure à celle d'un bon anticorps thérapeutique.

CRM197 est très immunogène. Elle ne diffère de la toxine diphtérique que par un résidu (G52E). Elle porte donc la totalité des épitopes T CD4 de la toxine diphtérique, ou presque, si la mutation affecte un épitope T. Or, les populations occidentales sont vaccinées contre la toxine diphtérique. Traiter des patients avec CRM197 revient à faire un rappel de vaccination. Dès la première injection, CRM197 peut entraîner la réactivation des cellules T CD4 mémoires et restimuler la production d'anticorps circulants. Quelques jours après la première administration de CRM197, ces anticorps devraient neutraliser la protéine et rendre le traitement inefficace.

CRM197 est très antigénique puisqu'elle possède virtuellement tous les épitopes B de la toxine diphtérique. La population occidentale étant vaccinée contre la toxine diphtérique, une fraction significative des individus possèdent des anticorps circulants capables de neutraliser CRM197 dès la première administration. Ceci impose d'utiliser des doses massives de CRM197 pour obtenir un effet thérapeutique, en augmentant considérablement les risques d'effets secondaires (toxicité, choc anaphylactique, etc.)

En conséquence, il existe un réel besoin de disposer de nouveaux inhibiteurs de l'HB-EGF qui possèdent une plus petite taille, une meilleure affinité pour l'HB-EGF, une antigénicité, une immunogénicité et une toxicité réduites, comparativement à CRM197.

Jusqu'à présent, il n'a pas été possible de produire le domaine DTR isolé, directement, sous forme recombinante, avec un rendement élevé, et sans utiliser de détergents ou d'agents chaotropiques ou dénaturants pour extraire la protéine recombinante des cellules transformées.

En effet, pour stabiliser la structure du domaine DTR isolé, et améliorer l'extraction et la purification de ce domaine à partir des bactéries transformées, il a été indispensable de fusionner le domaine DTR à une protéine (Gluthation-S-transférase, GST) ou à un domaine de protéine (domaine ZZ issu de la protéine A de *S. aureus* (Lobeck et al., Infect. Immun., 1998, 66, 418-423; Shen et al., J. Biol. Chem., 1994, 269, 29077-29084). En outre, des mutants de la boucle de la région 516-530 de DTR ont été produits à partir de la fusion GST-DTR ; ces mutants de DTR sont incapables de se lier au récepteur pro-HB-EGF (Shen *et al*., 1994).

Dans les deux cas précités, la protéine recombinante produite dans *E.coli* n'est pas un domaine DTR isolé mais une protéine de fusion GST-DTR ou ZZ-DTR. Dans le cas de la protéine de fusion GST-DTR les rendements de production sont médiocres et il est indispensable d'utiliser un détergent (1 % Triton X-100) pour extraire la protéine de fusion des bactéries transformées De plus, pour produire une protéine thérapeutique, le partenaire de fusion doit être éliminé, ce qui rend le procédé de production beaucoup plus complexe et diminue encore plus les rendements.

Les inventeurs ont construits des mutants du domaine DTR isolé, dépourvus de séquences d'un partenaire de fusion. Ces mutants du domaine DTR isolé s'expriment directement et en grande quantité sous forme d'une protéine DTR recombinante soluble, de petite taille (environ 17500 Da) affine pour le pro-HB-EGF et l'HB-EGF. Ces mutants de DTR ont été modifiés pour produire des protéines DTR recombinantes améliorées, qui de manière surprenante, possèdent, à la fois une antigénicité et une immunogénicité réduites et une affinité considérablement augmentée, comparativement à CRM197.

En conséquence, la présente invention a pour objet une protéine recombinante comprenant un domaine R isolé de la toxine diphtérique comprenant une séquence d'acides aminés ayant au moins 70 % d'identité avec les résidus 380 à 535 de la séquence d'acides aminés SEQ ID NO : 1 correspondants au domaine R de la toxine diphtérique, ladite séquence comprenant la combinaison de substitutions Y380K et L390T, et ladite séquence étant dépourvue à l'extrémité N- ou C-terminale dudit domaine R, de la séquence du domaine C et du domaine T de la toxine diphtérique et de la séquence d'une protéine ou d'un domaine de protéine capable d'améliorer la stabilité ou la purification dudit domaine R, et ladite protéine recombinante étant un ligand inhibiteur de l'HB-EGF.

L'invention est telle que définie dans les revendications.

L'invention fournit une protéine recombinante thérapeutique ligand de l'HB-EGF et du pro-HB-EGF qui présente les avantages suivants :
- elle a une solubilité très augmentée par rapport à la forme sauvage DTR_{WT}. La combinaison des substitutions Y380K et L390T, permet d'augmenter considérablement la solubilité de la protéine DTR en solution aqueuse. Ainsi, la protéine recombinante selon l'invention peut être extraite des cellules hôtes et purifiée sans utiliser de détergents ou d'agents chaotropiques ou dénaturants. Par comparaison, la protéine DTR sauvage (DTR_{WT}) produite dans le même système d'expression est insoluble et non solubilisée à l'aide de détergents compatibles avec une utilisation thérapeutique. La protéine DTR_{WT} est solubilisée en présence de 0,5 % de sarkosyl ou de sodium dodécyl sulfate, des détergents incompatibles avec une utilisation thérapeutique à ces concentrations.
- elle est beaucoup plus facile à produire sous forme recombinante que DTR_{WT}. Elle est produite directement, sans utiliser de partenaire de fusion pour améliorer la stabilité ou la purification du domaine R. Elle est produite chez *E. coli* selon des procédures de fermentation standard, sous une forme soluble, repliée, et en grande quantité après purification finale (plusieurs dizaines de mg/L de culture en conditions de laboratoire non optimisées).
- elle a une affinité pour HB-EGF et proHB-EGF au moins 10 fois supérieure à celle de CRM197. De manière surprenante, alors que la protéine DTR sauvage (DTR_{WT}) a une affinité pour HB-EGF et proHB-EGF qui est inférieure d'un facteur 2 à celle de CRM197, les protéines DTR mutantes selon l'invention ont une affinité pour HB-EGF et pro-HB-EGF considérablement supérieure à celle de CRM197 ; les protéines dénommées DTR1, DTR3 et DTR8 ont respectivement une affinité 60, 300 et 1400 fois supérieure à celle de CRM197. Cela signifie que DTR8 pourrait être utilisé à des doses très inférieures à CRM197 pour un même effet thérapeutique. De ce fait, les risques d'effets secondaires, liés en général à l'existence de liaisons de faible affinité avec d'autres protéines ou ligands de l'organisme, sont donc considérablement diminués par comparaison avec CRM197. Ces interactions sont supprimées par l'utilisation de doses faibles, de l'ordre du pM, ce qui devrait être le cas pour DTR8.
- elle a une immunogénicité diminuée par rapport à CRM197 et à DTR_{WT}. Dix des 26 épitopes T identifiés dans DTR_{WT} ont été supprimés de la protéine DTR8 par mutagénèse dirigée, dont 7 parmi 9 épitopes immunodominants.
- elle a une antigénicité très diminuée par rapport à CRM197 et à DTR_{WT}. Les sérums d'individus vaccinés contre la toxine diphtérique reconnaissent préférentiellement le domaine catalytique de celle-ci. Ce domaine est présent dans la molécule CRM197 et absent de la protéine DTR mutée selon l'invention. Par ailleurs, la protéine DTR mutée selon l'invention est moins bien reconnue que DTR_{WT} par les anticorps de sujets vaccinés qui reconnaissent DTR_{WT}.
- elle bloque une voie d'activation de l'EGFR (ErbB1 et ErbB4) par l'HB-EFG de manière beaucoup plus spécifique que les inhibiteurs commerciaux de l'EGFR (petites molécules et anticorps thérapeutiques), présentant de ce fait, potentiellement, beaucoup moins de risques d'effets secondaires,
- elle a une petite taille ; les protéines de SEQ ID NO : 2 à 5 et SEQ ID NO : 7 à 9 ont une séquence de 158 acides aminés et un PM d'environ 17500 Da, c'est-à-dire une taille 3,4 fois inférieure à celle de CRM197 et 8,8 fois inférieure à celle des anticorps anti-HB-EGF utilisés actuellement dans des protocoles cliniques pour bloquer la voie HB-EGF. Du fait de sa petite taille, la protéine recombinante selon l'invention est plus efficace dans le traitement des pathologies liées à l'activation de la voie de l'HB-EGF, en particulier la GNRP ou le cancer de l'ovaire, dans la mesure où elle diffuse plus facilement dans les tissus, notamment les tumeurs et qu'elle est capable de pénétrer dans les glomérules rénaux.

En outre, du fait de son affinité élevée pour HB-EGF et pro-HB-EGF, la protéine selon l'invention peut être également utilisée pour le diagnostic des maladies impliquant l'activation de la voie de l'HB-EGF/EGFR.

### Définitions

- Dans la présente demande, on entend par « DTR » ou « domaine DTR », le domaine R de la toxine diphtérique qui correspond aux résidus 380-385 à 531-535 de la séquence d'acides aminés de la toxine diphtérique sauvage (SEQ ID NO : 1).
- L'expression « protéine DTR » désigne une protéine recombinante comprenant un domaine DTR isolé, *i.e*., dépourvu à son extrémité N- ou C-terminale de la séquence du domaine C et du domaine T de la toxine diphtérique et de la séquence d'une protéine ou d'un domaine de protéine capable d'améliorer la stabilité ou la purification dudit domaine R. En outre, la purification du domaine R inclut l'extraction et la purification dudit domaine, étant donné qu'il est produit sous forme d'une protéine recombinante.
- La protéine recombinante selon l'invention qui comprend au moins deux substitutions telles que définies ci-dessus est désignée sous le nom de protéine DTR mutante, protéine DTR mutée ou protéine DTRₙ où n est un nombre entier, par opposition à la protéine recombinante DTR sauvage (DTR_{WT}) qui ne comprend pas cette substitution.
- Les acides aminés sont désignés à l'aide du code à une lettre.
- La similarité d'une séquence d'acides aminés par rapport à une séquence de référence s'apprécie en fonction du pourcentage de résidus d'acides aminés qui sont identiques ou qui différent par des substitutions conservatives, lorsque les deux séquences sont alignées de manière à obtenir le maximum de correspondance entre elles. Lorsque seuls les résidus identiques sont pris en compte et que le pourcentage de résidus identiques est déterminé, on parle alors de l'identité de ladite séquence d'acides aminés par rapport à la séquence de référence. Au sens de la présente Invention, on entend par substitution conservative dans la séquence d'acides aminés d'une protéine, la substitution d'un acide aminé par un autre acide aminé naturel ou synthétique qui présente des propriétés chimiques ou physiques similaires (taille, charge ou polarité), qui n'ont pas d'effet délétère sur l'activité biologique de la protéine. Ainsi deux séquences d'acides aminés d'une protéine sont similaires lorsqu'elles diffèrent entre elles par la substitution d'un acide aminé, la délétion et/ou l'insertion d'un acide aminé ou d'un nombre réduit d'acides aminés (inférieur à 5) à des positions qui n'ont pas d'effet délétère sur l'activité biologique de ladite protéine. Le pourcentage de similarité ou d'identité peut être calculé par l'Homme du métier en utilisant un logiciel de comparaison de séquences tel que, par exemple celui de la suite logicielle BLAST (Altschul et al., NAR, 1997, 25, 3389-3402). Les programmes BLAST sont mis en oeuvre en utilisant les paramètres par défaut, sur une fenêtre de comparaison constituée par les résidus 380 à 535 de la séquence d'acides aminés SEQ ID NO : 1.
- Sauf indication contraire le terme « HB-EGF » désigne à la fois le précurseur membranaire (pro-HB-EGF) et la forme sécrétée de l'HB-EGF.
- L'activité de ligand inhibiteur de l'HB-EGF se réfère à la capacité de liaison au pro-HB-EGF et à l'HB-EGF et à l'inhibition de différents phénomènes biologiques mesurables, tels que :
- l'inhibition de l'effet toxique de la toxine diphtérique sur des cellules humaines ou simiennes telles que des cellules Véro,
- l'inhibition de l'activité proliférante de l'HB-EGF sur des cellules exprimant les sous-types ErbB1 et/ou ErbB4 de l'EGFR et dont la croissance est dépendante de l'HB-EGF, telle qu'une lignée de cellules lymphoïdes murines Ba/F3 transfectée avec le gène de l'EGFR.

La protéine recombinante selon l'invention comprend un domaine DTR isolé, dépourvu à son extrémité N- ou C-terminale de l'une des séquences suivantes : (1) la séquence du domaine C et du domaine T de la toxine diphtérique, et (2) la séquence d'un partenaire de fusion capable d'améliorer la stabilité du domaine DTR, telle que la séquence de la Gluthation-S-Transférase (GST) ou capable d'améliorer l'extraction et la purification du domaine DTR à partir des bactéries transformées, telle que la séquence d'un domaine de liaison des immunoglobulines issu de la protéine A de *S. aureus* (domaine ZZ).

La protéine recombinante selon l'invention comprend généralement un domaine DTR isolé qui s'étend des résidus 380 à 535 de la séquence SEQ ID NO : 1 et comprend la combinaison des substitutions Y380K et L390T. Toutefois, elle peut également comprendre un domaine DTR isolé légèrement plus court dont l'extrémité N-terminale est en position 381 à 385 et l'extrémité C-terminale en position 531 à 534 de la séquence SEQ ID NO : 1. Lorsque la séquence N-terminale de DTR est en position 381 ou 382, la protéine recombinante selon l'invention comprend la substitution du résidu L390. Lorsque la séquence N-terminale de DTR est en position 383, 384 ou 385, la protéine recombinante selon l'invention comprend la substitution du résidu L390.

La protéine recombinante selon l'invention comprend éventuellement une méthionine (M) à son extrémité N-ter et/ou des séquences additionnelles en N- et/ou C-terminal du domaine DTR. En effet, le précurseur de ladite protéine recombinante comprend une méthionine N-terminale qui peut être clivée par des modifications post-traductionnelles, de sorte qu'elle est absente dans la protéine recombinante mature.

Pour des applications thérapeutiques, la protéine selon l'invention est constituée successivement de : (1) une séquence de 1 à 10 acides aminés, de préférence 1 à 5 acides aminés, de manière préférée de 1 ou 2 acides aminés, la séquence du précurseur et éventuellement celle de la protéine mature commençant par une méthionine (M), par exemple une séquence MG, et (2) un domaine DTR muté tel que défini ci-dessus. Une telle protéine thérapeutique a une petite taille, d'environ 145 à 200 acides aminés, de préférence d'environ 145 à 175 acides aminés, de manière préférée, d'environ 160 acides aminés.

Pour des applications diagnostiques, la protéine selon l'invention est marquée, notamment par un traceur peptidique qui peut être détecté par mesure d'un signal approprié. Le traceur peptidique est notamment une étiquette reconnue par un anticorps, une protéine fluorescente, ou un peptide qui lie au moins un atome de Technétium 99. Le marqueur est en N-ter ou C-ter de la protéine, *i.e*., fusionné, directement ou par l'intermédiaire d'un espaceur peptidique, respectivement à l'extrémité N-ter ou C-ter du domaine DTR. Une telle protéine a une taille d'environ 145 à 500 acides aminés, de préférence d'environ 145 à 300 acides aminés, de manière préférée d'environ 145 à 200 acides aminés.

L'invention englobe les protéines recombinantes modifiées dérivées de la précédente par l'introduction de toute modification au niveau de résidu(s) d'acide(s) aminé(s), de la liaison peptidique ou des extrémités de la protéine recombinante, dès lors que ladite protéine modifiée conserve une bonne affinité et une activité inhibitrice vis-à-vis de l'HB-EGF et du pro-HB-EGF. Ces modifications qui sont introduites dans les protéines par les méthodes classiques connues de l'Homme du métier, incluent de façon non-limitative : la substitution d'un acide aminé par un acide aminé synthétique (acide aminé D ou analogue d'acide aminé) ; l'addition de groupement chimique (lipide, oligo ou polysaccharide) au niveau d'une fonction réactive, notamment de la chaîne latérale R ; la modification de la liaison peptidique (-CO-NH-), notamment par une liaison du type rétro ou rétro-inverso (-NH-CO-) ou une liaison différente de la liaison peptidique ; la cyclisation ; la fusion (par génie génétique) à un peptide ou une protéine d'intérêt ou le couplage (par liaison chimique) à une molécule d'intérêt, notamment un agent de marquage ou traceur détectable par mesure d'un signal.

Ladite protéine comprend au moins les substitutions Y380K et L390T. Par exemple, ladite protéine comprend l'une des combinaisons de substitutions suivantes : Y380K et L390T ; Y380K, Q387E et L390T ; Y380K, Q387E, P388T et L390T ; Y380K, Q387E, P382T et L390T. De préférence, il s'agit d'une protéine qui comprend ou consiste en l'une des séquences SEQ ID NO : 2 à 5.

De manière encore plus préférée, ladite protéine comprend les substitutions Y380K, Q387E et L390T. Par exemple, ladite protéine comprend ou consiste en l'une des séquences SEQ ID NO : 3 à 5, de préférence la séquence SEQ ID NO : 3.

Selon un autre mode de réalisation avantageux de ladite protéine, elle comprend de plus la substitution A395T. L'ajout de la substitution produit une augmentation supplémentaire du rendement en protéine soluble. De préférence, ladite protéine comprend la séquence SEQ ID NO : 7 ; cette protéine dénommée DTR1 comprend les substitutions Y380K, Q387E, L390T et A395T. L'affinité de liaison de la protéine DTR1 au HB-EGF est augmentée d'un facteur 60 comparée à celle de CRM197 et d'un facteur 130 comparée à celle de la protéine DTR_{WT} solubilisée par 0,5% de détergent sarkosyl.

Selon encore un autre mode de réalisation avantageux de ladite protéine, elle comprend en outre, au moins l'une des substitutions F389Y et/ou G510A. L'ajout de l'une de ces substitutions augmente l'affinité de liaison de la protéine DTR au HB-EGF. En outre, la combinaison des deux substitutions produit une augmentation supplémentaire de l'affinité de liaison au HB-EGF, par rapport à l'augmentation obtenue avec une seule substitution. De préférence, ladite protéine comprend ou consiste en la séquence SEQ ID NO : 8 ; cette protéine dénommée DTR3 comprend les substitutions Y380K, Q387E, L390T, A395T, F389Y et G510A. L'affinité de la protéine DTR3 pour l'HB-EGF est augmentée d'un facteur 5 comparée à celle de DTR1 et d'un facteur 300 comparée à celle de CRM197.

Selon un autre mode de réalisation avantageux de ladite protéine, elle comprend également au moins une substitution sélectionnée dans le groupe constitué par : N399K, V452T, T517E, V483Q, H492E, S494K, T436H et E497D. La dite protéine comprend avantageusement au moins 3, de préférence au moins 5 desdites substitutions.

De préférence, ladite protéine comprend les substitutions N399K, V452T, T517E, V483Q, H492E et S494K. Ces mutations ont permis de supprimer 10 des 26 épitopes T CD4 identifiés dans DTR_{WT}. Parmi ces 10 épitopes se trouvent 7 des 9 épitopes prédits comme des épitopes immunodominants de DTR_{WT}. La capacité de la protéine résultante, d'induire une réponse immunitaire de type CD4, c'est-à-dire productrice d'anticorps, se trouve ainsi considérablement diminuée par rapport à celle de DTR_{WT}. De façon remarquable et inattendue, l'ajout des six mutations destinées à réduire l'immunogénicité de DTR a contribué à augmenter son affinité pour HB-EGF.

De manière préférée, ladite protéine comprend ou consiste en la séquence SEQ ID NO : 9 ; cette protéine dénommée DTR8 comprend les substitutions Y380K, Q387E, L390T, A395T, F389Y, G510A, N399K, V452T, T517E, V483Q, H492E et S494K. Elle a un poids moléculaire de 17458 Da.

L'affinité de la protéine DTR8 pour l'HB-EGF est augmentée d'un facteur 3 comparée à celle de DTR3 et d'un facteur 1400 comparée à celle de CRM197. En outre, DTR8 est au moins 300 fois plus efficace que CRM197 pour se lier à l'HB-EGF sécrétée et inhiber la voie HB-EGF/EGFR.

L'antigénicité de la protéine DTR8 se trouve très réduite par rapport à celle de CRM197. En effet, l'essentiel des anticorps présents chez des adultes vaccinés contre la DT (vaccination obligatoire) sont dirigés contre le domaine catalytique de la DT. De manière surprenante et inattendue, l'antigénicité de la protéine DTR8 se trouve réduite par rapport à celle de DTR1, et donc de DTR_{WT}.

La protéine selon l'invention peut comprendre des substitutions additionnelles, en particulier de résidus situés à la surface du domaine DTR, de manière à réduire encore d'avantage son antigénicité.

Selon un autre mode de réalisation avantageux de ladite protéine, elle comprend une séquence d'acides aminés qui a au moins 80 %, 85 % ou 90% d'identité, avec les résidus 380 à 535 de la séquence SEQ ID NO : 1.

Selon un autre mode de réalisation avantageux de ladite protéine, elle est marquée par un traceur détectable. Les moyens et les techniques de marquage des protéines sont bien connus de l'homme du métier et incluent le marquage radioactif, magnétique ou fluorescent qui peut être effectué directement ou indirectement. Les agents de marquage direct sont notamment des isotopes radioactifs tels que le tritium (³H), l'iode (¹²⁵I) et le technetium (^{99m}Tc) ou des composés luminescents (radioluminescents, chémoluminescents, bioluminescents, fluorescents ou phosphorescents) tels que les fluorophores comme de façon non-limitative AlexaFluor®, FITC et Cyanine 3, et les protéines fluorescentes telles que la GFP et ses dérivées. Les agents de marquage indirect incluent notamment les enzymes et les étiquettes épitopiques reconnues par un anticorps spécifique. Le marquage est notamment réalisé par : (1) greffage d'un fluorophore sur un groupement réactionnel tel qu'une amine réactionnelle d'un résidu de lysine, (2) incorporation d'un groupement réactionnel (cystéine libre) par synthèse chimique ou production recombinante, puis utilisation de ce groupement pour greffer un fluorophore, (3) incorporation directe d'un fluorophore, en N ou C-terminal, par synthèse chimique, (4) incorporation directe d'une protéine fluorescente ou d'une enzyme par production d'une protéine de fusion. De telles protéines marquées sont notamment utilisées comme réactif pour le diagnostic *in vitro* ou *in vivo* (par imagerie) des maladies liées à l'activation de la voie HB-EGF/EGFR ou comme outil pour l'étude de cette voie d'activation. La protéine DTR peut être marquée directement par couplage covalent de technetium (^{99m}Tc) ou d'un traceur fluorescent ; le traceur fluorescent est notamment couplé sur un des résidus de lysine de la protéine DTR.

La protéine recombinante de la présente invention peut être produite par un procédé dans lequel un vecteur d'expression comprenant un polynucléotide codant ladite protéine lié de manière fonctionnelle aux éléments régulateurs permettant son expression dans l'hôte choisi, est transféré dans une cellule hôte qui est mise en culture dans des conditions permettant l'expression de ladite protéine. La protéine produite est ensuite récupérée et purifiée. Les procédés de purification utilisés sont connus de l'homme du métier. La protéine recombinante obtenue peut être purifiée à partir de lysats et d'extraits cellulaires, du surnageant du milieu de culture, par des méthodes utilisées individuellement ou en combinaison, telles que le fractionnement, les méthodes de chromatographie, les techniques d'immuno-affinité à l'aide d'anticorps mono-ou polyclonaux spécifiques. La protéine recombinante obtenue est soluble.

La présente invention a également pour objet un polynucléotide isolé codant pour ladite protéine recombinante. Ledit polynucléotide comprend avantageusement une séquence codante optimisée pour l'expression chez l'hôte cellulaire dans lequel la protéine de l'invention est produite. De préférence ledit polynucléotide comprend la séquence SEQ ID NO : 10, 12 ou 14 qui est optimisée pour l'expression chez *E.coli* et code respectivement pour la protéine recombinante DTR1, DTR3 et DTR8. Le polynucléotide de l'invention est un ADN ou un ARN préparé par les méthodes classiques de synthèse chimique ou de biologie moléculaire connues de l'homme du métier.

La présente invention a également pour objet un vecteur recombinant de clonage et/ou d'expression comprenant ledit polynucléotide. De préférence, ledit vecteur est un vecteur d'expression comprenant ledit polynucléotide lié de manière fonctionnelle aux séquences régulatrices permettant l'expression de la protéine de l'invention chez l'hôte cellulaire utilisé pour la production de ladite protéine (promoteur, activateur, codon d'initiation (ATG), codon stop, signal de terminaison de transcription). Le vecteur, réplicatif ou intégratif, notamment un plasmide ou un vecteur viral est préparé selon les méthodes couramment utilisées par l'homme du métier.

La présente invention a également pour objet une cellule hôte modifiée de manière transitoire ou stable par un vecteur recombinant tel que défini ci-dessus. Ces cellules peuvent être obtenues par l'introduction dans une cellule hôte eucaryote ou procaryote d'un vecteur recombinant tel que défini ci-dessus, par des méthodes standards connues de l'homme du métier, telles que l'électroporation. Des exemples de cellules hôtes incluent notamment des cellules de mammifères, d'insectes, des bactéries telles que *E.coli* et des levures.

La présente invention a également pour objet une composition pharmaceutique, comprenant au moins une protéine recombinante ou un vecteur recombinant d'expression tels que définis ci-dessus et un véhicule pharmaceutiquement acceptable.

La composition pharmaceutique comprend une dose efficace de protéine ou de vecteur permettant d'obtenir un effet thérapeutique sur des maladies associées à une activation de la voie HB-EGF/EGFR telles que définies ci-dessus. De manière générale, une quantité thérapeutiquement efficace variant d'environ 0,1 µg à environ 100 mg, de préférence de 10 µg à 10 mg, peut être administrée à des adultes humains. Les véhicules pharmaceutiquement acceptables sont ceux classiquement utilisés. La composition se présente sous une forme galénique adaptée à une administration choisie : solution stérile injectable, poudre, comprimés, gélules, suspension, sirop, suppositoires, qui sont préparés selon les protocoles standards. L'administration peut être sous-cutanée, intramusculaire, intraveineuse, intradermique, intrapéritonéale, orale, sublinguale, rectale, vaginale, intranasale, par inhalation ou par application transdermique.

Les modes d'administration, les posologies et les formes galéniques des compositions pharmaceutiques selon l'invention peuvent être déterminées de manière usuelle par l'homme du métier, notamment selon les critères généralement pris en compte pour l'établissement d'un traitement thérapeutique adapté à un patient (généralement un individu humain et éventuellement un mammifère non-humain), comme par exemple l'âge, le poids corporel, la gravité de son état général, la tolérance du traitement et les effets secondaires constatés.

La présente invention a également pour objet une protéine recombinante telle que définie ci-dessus comme médicament.

La présente invention concerne également une protéine recombinante telle que définie ci-dessus pour utilisation dans le traitement de maladies liées à une activation de la voie HB-EGF/EGFR.

La présente invention a également pour objet l'utilisation d'une protéine recombinante telle que définie ci-dessus pour la préparation d'un médicament destiné au traitement de maladies associées à une activation de la voie HB-EGF/EGFR.

La présente invention a également pour objet une méthode de traitement de maladies liées à une activation de la voie HB-EGF/EGFR, comprenant l'administration d'une composition pharmaceutique telle que définie ci-dessus, à un individu. L'administration est réalisée selon un mode et un rythme appropriés tels que définis ci-dessus.

De préférence lesdites maladies sont sélectionnées dans le groupe constitué par : les glomérulonéphrites rapidement progressives (GNRP), les cancers, notamment le cancer de l'ovaire, de l'endomètre, du sein, de l'utérus (adénocarcinome utérin), de la vessie, de l'estomac, de la peau (mélanome malin), du cerveau (glioblastome) et du poumon, le vasospasme associé aux contusions cérébrales, l'hypertrophie cardiaque, l'hyperplasie des cellules musculaires lisses, l'hypertension pulmonaire, les rétinopathies diabétiques et la resténose artérielle.

La présente invention a également pour objet l'utilisation d'une protéine marquée telle que définie ci-dessus pour le diagnostic *in vitro* ou *in vivo* d'une maladie liée à une activation de la voie de l'HB-EGF/EGFR.

L'invention concerne également une méthode de diagnostic *in vitro* d'une maladie liée à une activation de la voie de l'HB-EGF/EGFR, comprenant la mise en contact d'un échantillon biologique avec une protéine marquée telle que définie ci-dessus dans des conditions permettant la formation d'un complexe spécifique entre ladite protéine marquée et l'HB-EGF et/ou le proHB-EGF, et la détection desdits complexes protéine/HB-EGF marqués, par tout moyen approprié.

L'échantillon biologique est notamment un échantillon de biopsie (rein, tumeur, muscle lisse, coeur, poumon, vaisseaux, rétine), du sérum ou de l'urine.

La présente invention a également pour objet l'utilisation d'une protéine marquée telle que définie ci-dessus pour la détection, *in vitro* ou *in vivo*, de l'HB-EGF.

La présente invention a également pour objet un procédé de détection de l'HB-EGF, *in vitro* et *in vivo*, comprenant au moins les étapes suivantes :
- la mise en contact de cellules à analyser avec une protéine marquée telle que définie ci-dessus, et
- la détection des cellules et/ou du milieu extracellulaire marqués, par tout moyen approprié.

Ce procédé permet de déterminer le profil d'expression tissulaire de l'HB-EGF dans des conditions physiologiques, pathologiques ou en réponse à un stimulus endogène ou exogène. La détection de l'HB-EGF, *in vivo*, dans l'organisme d'un mammifère (imagerie cellulaire), notamment en temps réel, comprend une étape préalable d'administration de ladite protéine audit mammifère (injection parentérale, administration orale). La détection de l'HB-EGF *in vivo* chez l'homme peut être utilisée pour diagnostiquer une maladie liée à une activation de la voie de l'HB-EGF/EGFR.

Le marquage des cellules et du milieu extracellulaire contenant de l'HB-EGF est notamment un marquage fluorescent, radioactif, ou un marquage magnétique, détectable par toute technique connue de l'Homme du métier (microscopie de fluorescence, cytométrie de flux, gammagraphie, imagerie de résonance magnétique).

L'invention a également pour objet un kit pour la mise en oeuvre des méthodes de diagnostic ou de détection telles que définies ci-dessus, comprenant une protéine marquée telle que définie ci-dessus.

Outre les dispositions qui précèdent, l'invention comprend encore d'autres dispositions, qui ressortiront de la description qui va suivre, qui se réfère à des exemples de mise en oeuvre de l'objet de la présente invention, avec référence aux dessins annexés dans lesquels :
- la figure 1 représente les mutations (A) et l'analyse (B) des fractions solubles et insolubles des 5 clones sélectionnés par criblage de la banque R1 pour produire une protéine DTR plus soluble que DTR_{WT}. Tous les clones sont solubles ; le meilleur clone est le clone 1D6.
- la figure 2 montre les effets des mutations A395T et N399D sur la solubilité de la protéine DTR produite par le clone 1D6 (ici noté G1). Le meilleur clone contient la mutation A395T.
- la figure 3 représente : (A) l'inhibition de l'effet toxique de doses croissantes de toxine diphtérique sur des cellules Vero par des doses croissantes de DTR1et (B) la régression de Schild permettant d'évaluer le Kd de DTR1 pour HB-EGF selon l'équation log (*EC₅₀* - 1) = *K_{d}* log (*B*) où *EC₅₀* est la concentration de toxine diphtérique donnant 50% de toxicité et *B* la concentration d'inhibiteur DTR1.
- la figure 4 représente l'inhibition de l'effet toxique de la toxine diphtérique à la concentration de 10⁻¹¹ M sur des cellules Vero par chaque mutant de DTR1 (ici noté G2) à une concentration de 10⁻⁹ M.
- la figure 5 représente : (A) l'inhibition de l'effet toxique de doses croissantes de toxine diphtérique sur des cellules Vero par des doses croissantes de DTR8 et (B) : la régression de Schild permettant d'évaluer le Kd de DTR8 pour HB-EGF selon l'équation log (*EC₅₀* - 1) = *K_{d}* log (*B*) où *EC₅₀* est la concentration de toxine diphtérique donnant 50% de toxicité et *B* la concentration d'inhibiteur DTR8.
- la figure 6 représente l'inhibition de l'effet prolifératif de doses croissantes de HB-EGF sur des cellules Ba/F3 exprimant l'EGFR et dépendante d'HB-EGF pour leur croissance, par des doses croissantes de DTR8 (A) et de CRM197 (B).
- la figure 7 représente la reconnaissance des protéines CRM197, domaine C (C) et domaine T (T) de la toxine diphtérique, DTR1 et DTR8 par les anticorps présents dans le sérum de 20 donneurs sains. Le titre est défini par la dilution de sérum donnant un signal de fluorescence de 5000 unités arbitraires dans le test ELISA, après soustraction du bruit de fond. Un titre ≤ 20 correspond au bruit de fond et donc à l'absence de liaison d'anticorps mesurable. Un titre de 30 a été fixé de manière arbitraire pour distinguer les réponses anticorps faibles de réponses moyennes ou fortes.

### Exemple 1 : Matériels et méthodes

### 1) Clonage des séquences génétiques, expression et purification de la protéine DTR_{WT} et de ses mutants

La séquence génétique codant le domaine DTR de la toxine diphtérique, recouvrant la séquence Y380-S535 de la toxine native a été le point de départ de cette étude. Toutes les séquences génétiques ont été synthétisées après optimisation pour une expression chez *E. coli* par la société GENEART d'après les séquences protéiques préalablement déterminées. Ses séquences ont été clonées dans le vecteur pET28a(+) (NOVAGEN) au niveau des sites de restriction *Nco*I et *Sal*I. La présence du site *Nco*I génère les codons non natifs M et G correspondant à l'extrémité N-terminale de la protéine recombinante. La séquence SEQ ID NO : 16 est la séquence nucléotidique optimisée qui code pour la protéine DTR sauvage (DTR_{WT}) de 158 acides aminés qui consiste aux résidus M et G suivis des résidus Y380 à S535 de la toxine diphtérique native. Les séquences nucléotidiques optimisées codant pour les formes mutantes et solubles de la protéine DTR dérivent de la séquence SEQ ID NO : 16 par le remplacement de chaque codon à muter par un codon optimisé codant pour l'acide aminé muté, comme indiqué dans le Tableau I :

**Tableau I : Liste des codons optimisés choisis pour les mutations de DTR**

| **Mutation** | **Codon sauvage** | **Codon muté optimisé** |
|---|---|---|
| Y380K | tac | aaa |
| Y380E | tac | gaa |
| P382T | ccg | acg |
| Q387E | cag | gag |
| Q387K | cag | aag |
| P388T | ccg | acg |
| F389Y | ttt | tat |
| L390T | ctg | acc |
| L390N | ctg | aac |
| H391K | cat | aaa |
| A395T | gcg | acc |
| N399D | aac | gat |
| N399K | aac | aaa |
| V401Q | gtg | cag |
| L427Q | ctg | cag |
| L427N | ctg | aac |
| L427S | ctg | agc |
| T436K | acc | aaa |
| T436H | acc | cat |
| V452T | gtg | acg |
| I457D | att | gat |
| I457E | att | gaa |
| R460T | cgt | acc |
| A463T | gcg | acc |
| A463S | gcg | agc |
| A463E | gcg | gaa |
| A463D | gcg | gat |
| A463G | gcg | ggc |
| Y478T | tat | acc |
| V483D | gtg | gat |
| V483E | gtg | gaa |
| V483H | gtg | cat |
| V483Q | gtg | cag |
| A490G | gcg | ggc |
| H492E | cat | gaa |
| S494K | agc | aaa |
| S496K | agc | aaa |
| E497D | gaa | gat |
| G510A | ggc | gcg |
| G510M | ggc | atg |
| G510Q | ggc | *cag* |
| G510S | ggc | agc |
| Q515E | cag | gaa |
| T517D | acc | gat |
| T517E | acc | gaa |
| T521R | acc | cgc |
| K522R | aaa | cgc |

L'expression de la protéine DTR_{WT} a été réalisée dans la bactérie *Escherichia coli* BL21(DE3) en milieu Terrific Broth en présence de 50 µg/mL de kanamycine à 37°C. L'induction de la protéine est réalisée par ajout de 1 mM d'isopropyl β-D-1-thiogalactopyranoside (IPTG). Les bactéries ont été récupérées par centrifugation à 5000g pendant 45 min et lysées dans un tampon 20 mM phosphate de sodium, 500 mM NaCl, 0,25 mM phenylmethylsulfonyl fluoride (PMSF), Lysozyme (0,25 mg/mL), pH 8 par passage au Cell Disrupter (CONSTANT SYSTEM). Les corps d'inclusion contenant la protéine ont été solubilisés dans une solution contenant 8 M d'urée en 0,1 M Tris-HCl, pH 8. La protéine est purifiée par chromatographie échangeuse de cation sur colonne HiTrap™ SP 5 mL (GE HEALTH CARE LIFE SCIENCES) selon un gradient de tampon entre 8 M d'urée en 0,1 M Tris-HCl, pH 8 et 8 M d'urée, 1 M NaCl en 0,1 M Tris-HCl, pH 8, puis repliée par dialyse en 2 étapes, premièrement contre un tampon 20 mM Tris-HCl, 50 mM NaCl, 1mM Cysteine/8mM Cystine, 0,5 % Sarkosyl, pH 8 puis contre un tampon 20 mM Tris-HCl, 50 mM NaCl, 0,5% Sarkozyl, pH 8.

L'expression des formes mutantes et solubles de la protéine DTR a été réalisée dans la bactérie *Escherichia coli* BL21(DE3) en milieu 2YT en présence de 50 µg/mL de kanamycine. Après 2 h 20 min de culture à 37°C, l'induction de la protéine est réalisée par ajout de 1 mM d'IPTG. Ensuite après 4 h 30 min à 30°C les bactéries ont été récupérées par centrifugation à 5000g pendant 30 min et lysées dans un tampon 20 mM phosphate de sodium, 0,25 mM PMSF, 2 mM MgCl2, additionné de Benzonase (6,25 U/mL), pH 7,8 au Cell Disrupter (CONSTANT SYSTEM). La protéine, soluble dans le mélange de lyse a été purifiée par chromatographie échangeuse de cation sur colonne HiTrap™ SP 5mL selon un gradient entre 20 mM phosphate de sodium pH 7,8 et 20 mM phosphate de sodium, 1 M NaCl pH 7,8 (GE) et stockée à -20°C dans un tampon 20 mM phosphate de sodium, 500 mM NaCl, pH 7,8.

### 2) Crible de sélection de mutants solubles

Trois banques de séquences de DTR réalisées par synthèse et clonées dans le plasmide pET28a(+) ont été transfectées dans la souche *E. coli* BL21(DE3) (GENEART). Pour chaque banque, des clones ont été repiqués dans 2 ou 4 plaques de 96 puits en milieu de culture en présence d'un antibiotique de sélection et d'inducteur d'expression (IPTG). Après croissance, les bactéries ont été lysées par une solution Bugbuster™ (0,5X final) (NOVAGEN) avec addition de Lysonase™ (NOVAGEN). Après centrifugation des plaques 20 min à 2700g à 4°C, la présence de corps d'inclusions a été évaluée puits par puits par la taille du culot et la fraction de protéine soluble a été identifiée par analyse des surnageants de lyse sur gel de polyacrylamide en condition dénaturante et coloration au bleu de Coomassie.

### 3) Test d'activité des protéines DTR par inhibition de la toxicité de la toxine diphtérique

Des cellules Vero (ATCC CCL-81™) ont été ensemencées en plaques 96 puits Cytostar-T™ (PERKIN ELMER) à fond scintillant à 50,000 cellules par puits en milieu D-MEM (**milieu** essentiel minimum de Eagle modifié par Dulbecco) additionné de 2 mM de glutamine, 1% d'une solution pénicilline/streptomycine, 1% d'une solution d'acides aminés non essentiels et de 10% de sérum de veau foetal. Des concentrations variables de toxine diphtérique (SIGMA) ont été ajoutées en dupliquas. Ces conditions ont été répétées en présence de l'antagoniste testé : protéine DTR_{WT}, DTR mutante ou CRM197 (Sigma). Après 22 h d'incubation à 37°C dans une atmosphère à 5% de CO₂, le milieu de culture a été remplacé par du milieu sans leucine contenant 1 µCi/puits de ¹⁴[C]-leucine (GE HEALTH CARE LIFE SCIENCES). Après 5 h d'incubation à 37°C dans une atmosphère à 5% de CO₂, la radioactivité incorporée dans les cellules a été comptée en plaçant les plaques recouvertes d'un film adhésif dans un appareil MicroBeta® (WALLAC).

### 4) Test d'activité des protéines DTR par inhibition de l'activité proliférante de l'HB-EGF

Le test utilise une lignée de cellules lymphoïdes murines Ba/F3 (Palacios, R & Steinmetz, M., Cell, 1985, 81, 727-734) transfectées au laboratoire avec le gène de l'EGFR. Cette lignée est dépendante de l'HB-EGF ou de l'Amphiréguline pour sa croissance. Les cellules ont été ensemencées en plaque 96 puits Nunclon™ (NUNC) à 10,000 cellules par puits en milieu RPMI (Roswell Park Memorial Institute medium) 1640 additionné de 2 mM de glutamine, 1% d'une solution pénicilline/streptomycine, 1% d'une solution d'acides aminés non essentiels et 10% de sérum de veau foetal. Des concentrations variables d'HB-EGF ou d'Amphiréguline ont été ajoutées en dupliquas. Ces conditions ont été répétées en présence de l'antagoniste testé : protéine DTR mutante ou CRM197. Après 24 h d'incubation à 37°C dans une atmosphère à 5% de CO₂, 1 µCi/puits de ³[H]-thymidine (GE HEALTH CARE LIFE SCIENCES) a été ajoutée à chaque puits. Après 5 h d'incubation à 37°C dans une atmosphère à 5% de CO₂, les cellules ont été aspirées sur un filtre de fibre de verre (filtermat A, WALLAC) à l'aide d'un appareil TOMTEC®. Après séchage des filtres, ceux-ci ont été placés dans un sachet scellé en présence de liquide à scintillation et la radioactivité incorporée dans les cellules a été comptée dans un appareil MicroBeta® (WALLAC).

### 5) Identification des épitopes T CD4 de la protéine DTR

L'identification des épitopes T CD4 de la protéine DTR est réalisée par l'analyse des peptides de DTR reconnus par des lignées lymphocytaires T CD4 spécifiques de DTR, restreintes aux molécules HLA-DRB1 prépondérantes dans les phénotypes caucasiens. Les protocoles utilisés sont ceux précédemment décrits dans la Demande WO 2010/076413 à l'exception des modifications suivantes : (1) les lignées lymphocytaires T CD4 spécifiques de DTR sont produites par co-culture de lymphocytes T CD4 du donneur avec des cellules dendritiques matures autologues chargées avec un pool de peptides chevauchants de DTR et (2) la spécificité des lignées produites est analysée par un test ELISPOT-IFN-γ en utilisant des cellules mononuclées du sang périphérique (PBMC) autologues chargées, soit avec le pool de peptides utilisé pour produire la lignée de lymphocytes T CD4, pour vérifier la spécificité de la lignée pour DTR, soit avec un peptide du pool, pour déterminer la spécificité de chaque lignée.

### a) Isolement des lymphocytes T CD4 à partir de PBMC

Sept donneurs d'âge et de phénotype HLA-DRB1 différents ont été sélectionnés de sorte que l'ensemble de ces donneurs exprime les 8 molécules HLA-DRB1 prépondérantes dans les phénotypes caucasiens (HLA-DR1 ; HLA-DR3 ; HLA-DR4 ; HLA-DR7 ; HLA-DR11 ; HLA-DR13 ; HLA-DR15 ; HLA-DR8). Les lymphocytes T CD4 de chaque donneur ont été isolés des cellules mononucléées du sang périphérique (PBMC), avec un degré de pureté supérieur à 98 %, par tri magnétique sur colonne à l'aide de billes magnétiques couplées à un anticorps anti-CD4, selon une procédure standard définie par le fabricant (MYLTENYI BIOTECH).

### b) Production et caractérisation des lignées lymphocytaires T CD4 spécifiques de DTR

25 peptides chevauchants de 15 acides aminés couvrant la séquence entière du DTR ont été synthétisés (INTAVIS BIOANALYTICAL INSTRUMENTS).

**Tableau II: Peptides chevauchants couvrant la séquence de DTR (SEQ ID NO : 18 à 42)**

| **Peptide** | **Localisation** | **Séquence en acides aminés** | | | | | | | | | | | | | | |
|---|---|---|---|---|---|---|---|---|---|---|---|---|---|---|---|---|
| **1** | **378-392** | M | G | Y | S | P | G | H | K | T | Q | P | F | L | H | D |
| **2** | **385-399** | K | T | Q | P | F | L | H | D | G | Y | A | V | S | W | N |
| **3** | **391-405** | H | D | G | Y | A | V | S | W | N | T | V | E | D | S | I |
| **4** | **397-411** | S | W | N | T | V | E | D | S | I | I | R | T | G | F | Q |
| **5** | **403-417** | D | S | I | I | R | T | G | F | Q | G | E | S | G | H | D |
| **6** | **409-423** | G | F | Q | G | E | S | G | H | D | I | K | I | T | A | E |
| **7** | **415-429** | G | H | D | I | K | I | T | A | E | N | T | P | L | P | I |
| **8** | **421-435** | T | A | E | N | T | P | L | P | I | A | G | V | L | L | P |
| **9** | **427-441** | L | P | I | A | G | V | L | L | P | T | I | P | G | K | L |
| **10** | **433-447** | L | L | P | T | I | P | G | K | L | D | V | N | K | S | K |
| **11** | **439-453** | G | K | L | D | V | N | K | S | K | T | H | I | S | V | N |
| **12** | **445-459** | K | S | K | T | H | I | S | V | N | G | R | K | I | R | M |
| **13** | **451-465** | S | V | N | G | R | K | I | R | M | R | C | R | A | I | D |
| **14** | **457-471** | I | R | M | R | C | R | A | I | D | G | D | V | T | F | C |
| **15** | **463-477** | A | I | D | G | D | V | T | F | C | R | P | K | S | P | V |
| **16** | **469-483** | T | F | C | R | P | K | S | P | V | Y | V | G | N | G | V |
| **17** | **475-489** | S | P | V | Y | V | G | N | G | V | H | A | N | L | H | V |
| **18** | **482-496** | G | V | H | A | N | L | H | V | A | F | H | R | S | S | S |
| **19** | **488-502** | H | V | A | F | H | R | S | S | S | E | K | I | H | S | N |
| **20** | **494-508** | S | S | S | E | K | I | H | S | N | E | I | S | S | D | S |
| **21** | **500-514** | H | S | N | E | I | S | S | D | S | I | G | V | L | G | Y |
| **22** | **506-520** | S | D | S | I | G | V | L | G | Y | Q | K | T | V | D | H |
| **23** | **512-526** | L | G | Y | Q | K | T | V | D | H | T | K | V | N | S | K |
| **24** | **518-532** | V | D | H | T | K | V | N | S | K | L | S | L | F | F | E |
| **25** | **521-535** | T | K | V | N | S | K | L | S | L | F | F | E | I | K | S |

Les peptides ont été regroupés en 3 pools :
- Pool 1 : peptides 1 à 8
- Pool 2 : peptides 9 à 16
- Pool 3 : peptides 17 à 25

Chacun des pools est utilisé *in vitro* pour sensibiliser indépendamment, de façon répétée, des lymphocytes T CD4 issus des donneurs sélectionnés pour l'étude. Un minimum de 30 puits de co-culture est réalisé par pool de peptides. Dans un premier temps, les cellules T CD4 sensibilisées capables de reconnaître le pool de peptides utilisé lors de la stimulation, appelées lignées lymphocytaires T CD4, sont sélectionnées par un test ELISPOT-IFN-γ en utilisant des PBMC autologues chargées avec le pool de peptides comme cellules présentatrices de l'antigène. Dans un second temps, le ou les peptides reconnus spécifiquement par les lignées T CD4 sélectionnées lors de la première analyse sont identifiés par un test ELISPOT-IFN-γ en utilisant des PBMC autologues chargés séparément avec chacun des peptides du pool comme cellules présentatrices de l'antigène. La spécificité de la reconnaissance de l'antigène (peptide isolé ou pool de peptides) est définie par : (1) un rapport entre le nombre de lymphocytes T CD4 productrices d'IFN-γ en réponse à l'antigène (PBMC + peptides), par rapport au bruit de fond (absence de l'antigène soit PBMC seuls) qui est supérieur à 2, et (2) un nombre minimum de 30 spots en présence d'antigène une fois le bruit de fond retranché.

### 6) Evaluation de l'antigénicité des protéines DTR

A chaque étape, les incubations ont été réalisées dans l'une ou l'autre des trois conditions suivantes : 1 h à 37°C ou 2 h à 20°C ou 16 h à 4°C. Les protéines testées (CRM197, domaine catalytique, domaine de translocation, DTR1 et DTR8) ont été solubilisées à la concentration de 1,7x10⁻⁸ M dans du tampon PBS à pH 7,4 pour être adsorbées sur des plaques 96 puits Maxisorp™ (NUNC). Les puits ont été saturés par une solution de sérum albumine bovine (BSA) (SIGMA) à 3% dans du PBS. Après 4 lavages avec une solution de tampon PBS contenant 0,05% de Tween 20, les sérums de donneurs sains ont été incubés en dupliquas dans les puits après dilution au 1/20^{ème}, 1/200^{ème} ou 1/2000^{ème} dans un tampon PBS contenant 0,2% de BSA et 0,05% de Tween 20. Après 4 lavages avec une solution de tampon PBS contenant 0,05% de Tween 20, un anticorps de chèvre anti-IgG humaines conjugué à la phosphatase alcaline (SIGMA) a été incubé dans les puits après dilution au 1/200^{ème} dans une solution de PBS contenant 0,2% de BSA et 0,05% de Tween 20. Après 4 lavages dans une solution de PBS contenant 0,05% de Tween 20, le test a été révélé par incubation dans une solution à 0,1 mM de 4-Methylumbelliferyl phosphate (SIGMA) diluée dans du tampon 50 mM carbonate, 1 mM MgCl₂ à pH 9,8 pendant 30 min à 20°C. La fluorescence des puits (émission à 450 nm) a été mesurée dans un fluorimètre Victor (WALLAC) par excitation à 365 nm.

Avant d'être testé, les sérums ont été mis à reposer à 20°C pendant une journée, centrifugés à 10.000g pendant 10 min puis additionnés de 0,003% de thimérosal avant stockage à 4°C ou à - 20°C.

### Exemple 2 : Amélioration de la solubilité de la protéine DTR

La protéine DTR_{WT} exprimée chez *E. coli* s'accumule en corps d'inclusion insolubles. La protéine n'a pu être obtenue, solubilisée et purifiée qu'en présence de 0,5% de sarkosyl ou de sodium dodécyl sulfate, des détergents incompatibles avec une utilisation thérapeutique à ces concentrations. L'utilisation d'autres molécules solubilisantes a été infructueuse (Tween-80, sucrose, arginine). L'utilisation d'agents chaotropiques tels l'urée ou le chlorure de guanidine pour solubiliser la protéine suivi d'une dialyse contre différents tampons de repliement ne permet pas non plus d'obtenir une protéine fonctionnelle soluble. L'influence du site de troncature de DTR par rapport à la séquence de la toxine diphtérique complète a également été étudiée. Les formes de DTR débutant au résidu A379, Y380, S381, P382, G383, H384, K385 étaient toutes insolubles en l'absence de détergent.

La stratégie employée pour augmenter la solubilité de la protéine DTR a consisté à muter les résidus hydrophobes présents à la surface de la protéine par des résidus hydrophiles, polaires ou chargés. En effet, les résidus hydrophobes à la surface d'une protéine sont potentiellement responsables d'une faible solubilité et d'une tendance à l'agrégation. Les mutations à introduire ont été identifiées sur la base d'une modélisation moléculaire. L'effet potentiel des mutations sur la structure de la protéine est indiqué dans le Tableau III.

**Tableau III : Effet escompté des mutations sélectionnées**

| **Positions** | **Mutations** | **Remarque d'ordre structural et interactions** |
|---|---|---|
| Y380 | - | flexible à la fin de la boucle N-ter, pas de liaison hydrogène stable |
| | Y380E | liaison ionique avec K385 |
| | Y380K | liaison ionique avec E532 |
| P382 | - | Dans un coude de type II |
| | P382T | |
| Q387 | - | Dans boucle N-ter, pas de liaison hydrogène stable |
| | Q387E | liaison ionique avec Y380K |
| P388 | - | Dans boucle N-ter, juste avant un brin béta |
| | P388T | P388T |
| L390 | - | Dans un brin béta |
| | L390N | donneur de liaison hydrogène pour le groupe CO du squelette de Y394 |
| | L390T | residu béta-branché favorisé |
| A395 | - | Dans un brin béta |
| | A395T | residu béta-branché favorisé |
| N399 | - | Dans une boucle, pas de liaison hydrogène stable |
| | N399D | liaison ionique avec K419 |
| N424 | - | Dans une boucle, pas de liaison hydrogène stable |
| | N424D | liaison ionique avec N481K |
| | N424E | liaison ionique avec N481K |
| | N424K | liaison ionique avec E423 ou N481E ou N481D |
| P426 | - | A l'extrémité d'une boucle |
| | P426T | |
| L427 | - | Dans un brin béta, contacts de Van der Waals avec Y394 |
| | L427K | Contacts de Van der Waals avec Y394, donneur de liaison hydrogène pour T425, liaison ionique avec E423 |
| | L427R | contacts de Van der Waals avec Y394, donneur de liaison hydrogène pour T425, liaison ionique avec E423 |
| P428 | - | Dans un renflement |
| | P428T | |
| P476 | - | Dans un renflement |
| | P476T | |
| Y478 | - | Dans un brin béta, contacts de Van der Waals avec P426, P428, P476 |
| | Y478D | |
| | Y478N | |
| N481 | - | Dans une boucle, pas de liaison hydrogène stable |
| | N481D | liaison ionique avec N424K |
| | N481E | liaison ionique avec N424K |
| | N481K | liaison ionique avec N424E ou N424D |
| V483 | - | Dans une boucle |
| | V483T | |

Trois banques de séquences d'ADN de DTR ont été réalisées par synthèse (Tableau IV). Chaque banque contenait des séquences mutées sur 4 ou 5 codons choisis en fonction des données de modélisation moléculaire. Chaque banque correspondait à des codons mutés dans une même région de la séquence codante. Les séquences contenaient des dégénérescences partielles des codons à muter de manière à limiter les mutations possibles à des résidus hydrophiles potentiellement acceptables selon les données de la modélisation (1, 2 ou 3 mutations possibles par position). La possibilité de conserver le codon sauvage a été maintenue, au cas où la position testée ne peut tolérer de mutation (Tableau IV), sauf pour Y380 dont la position N-terminale relativement peut contrainte a été considérée tolérante.

**Tableau IV: Combinaisons de mutations possibles attendues pour chacune des trois banques de séquences mutantes générées pour augmenter la solubilité du domaine R (R1, R2, R3).**

| **Région mutée** | **Positions mutées** | **Résidus possibles** | **Diversité de la banque** |
|---|---|---|---|
| R1 | Y380 | K/E | 72 |
| | P382 | P/T | |
| | Q387 | Q/E/K | |
| | P388 | P/T | |
| | L390 | L/T/N | |
| R2 | N424 | N/K/D/E | 48 |
| | P426 | P/T | |
| | L427 | L/R/K | |
| | P428 | P/T | |
| R3 | P476 | P/T | 48 |
| | Y478 | Y/N/D | |
| | N481 | N/K/D/E | |
| | V483 | V/T | |

| | | | |
|---|---|---|---|
| *La diversité indique le nombre de combinaisons de mutations possibles pour chacune des banques. | | | |

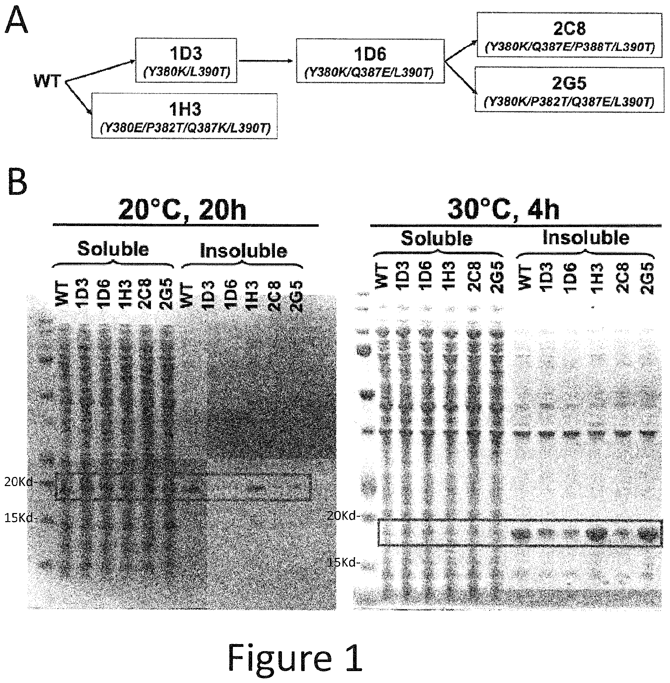

Après transfection des banques d'ADN, les colonies bactériennes obtenues correspondant chacune à une combinaison de mutations donnée ont été analysées pour leur capacité éventuelle à produire une forme mutante soluble de la protéine DTR. Pour cela, 740 clones issus des trois banques, représentant 85% de la diversité attendue (41 clones différents pour chaque banque), ont été repiqués en plaques à 96 puits, cultivés en conditions d'induction de l'expression de la protéine, centrifugés puis lysée dans une solution de lyse. Après centrifugation, la solubilité des protéines exprimées par chaque clone a été évaluée par observation d'un culot de corps d'inclusion éventuel au fond du puits et par analyse du surnageant par électrophorèse en gel de polyacrylamide en condition dénaturante et coloration au bleu de Coomassie (Figure 1).

L'analyse des colonies a conduit à sélectionner les clones présentant une absence de culot (culot correspondant aux corps d'inclusion) ou un culot de taille réduite ainsi qu'une forte quantité de protéine dans le surnageant de lyse. Seule la banque R1 a permis de générer des clones producteurs de DTR soluble (Figure 1), au nombre de cinq. Ces clones dénommés 1D3, 1D6, 2C8, 5G5 et 1H3 comprennent les séquences nucléotidiques codant pour les protéines SEQ ID NO : 2 à 6, respectivement. Tous les clones étaient solubles après purification selon la méthode décrite à l'exemple 1. Le clone 1D6, appelé aussi G1, permettant d'obtenir la plus grande quantité de DTR sous forme soluble après expression à 30°C portait les mutations : Y380K/Q387E/L390T.

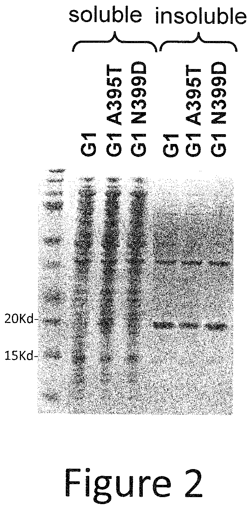

L'approche de modélisation a conduit à proposer deux mutations supplémentaires, non représentées dans la banque R1 : A395T et N399D. Ces deux mutations ont été chacune introduites dans le clone 1D6 (ou G1) pour rechercher un effet solubilisant augmenté, ce qui a été le cas pour la mutation A395T (Figure 2).

Au total, la protéine DTR la plus soluble, appelée DTR1 (SEQ ID NO : 7), porte les mutations : Y380K/Q387E/L390T/A395T

Ceci n'exclut pas que d'autres mutations de DTR puissent améliorer encore sa solubilité et sa production dans *E. coli.*

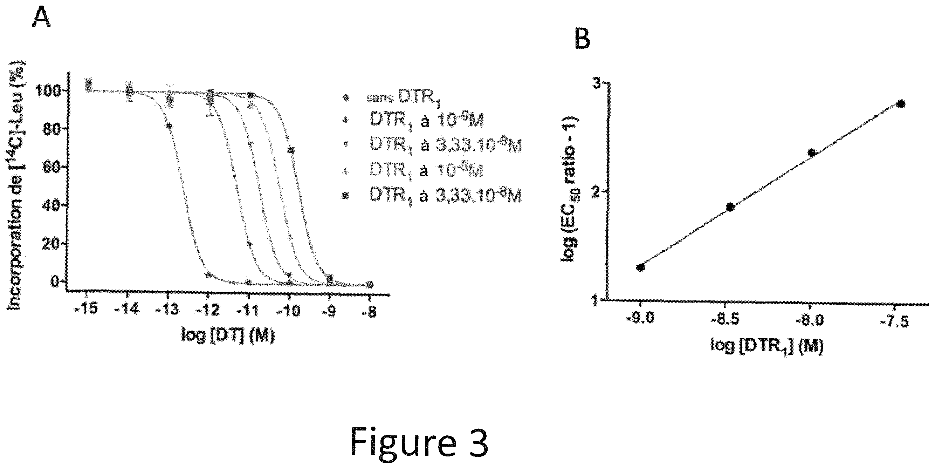

L'activité de liaison de la protéine DTR1 à HB-EGF a été évaluée par sa capacité à inhiber l'intoxication de cellules Vero par la toxine diphtérique, et donc la liaison de la toxine au pro-HB-EGF. Les résultats montrent que DTR1 inhibe l'intoxication de cellules Vero par la toxine diphtérique de manière dose-dépendante (Figure 3). L'effet inhibiteur de DTR1 a été comparé à celui de CRM197, du clone 1D6 et DTR_{WT} solubilisé dans 0,5% de sarkosyl. Les Kd estimés par régression de Schild pour les trois interactions donnent les valeurs suivantes :

**Tableau V : Affinité pour HB-EGF**

| **protéine** | **Kd (pM)** |
|---|---|
| DTR1 | ∼ 49 |
| Clone 1D6 | ∼25 |
| DTR_{WT} (+ 0,5% sarkosyl) | ∼ 6500 |
| CRM197 | ∼ 3100 |

Des expériences de Biacore dans lesquelles l'HB-EGF a été immobilisé sur la puce de l'appareil et DTR1 injecté dans la fraction mobile pour mesure des constantes d'association et de dissociation a permis de confirmer le Kd estimé de CRM197 et de montrer un Kd très supérieur pour DTR1. Cependant, la lenteur de la dissociation ne permet pas une mesure précise de la constante de dissociation de DTR1 en Biacore. Dans la suite de l'étude, l'affinité des mutants a été estimée par cytotoxicité et régression de Schild.

### Exemple 3 : Amélioration du site de liaison de la protéine DTR

La structure de la toxine diphtérique en interaction avec l'HB-EGF (Louie et al., Mol. Cell., 1997, 1, 67-78) permet d'analyser l'interface entre les deux protéines. Cette analyse structurale, couplée à des expériences *in silico* de modélisation moléculaire a permis de sélectionner 10 mutations dans le site de liaison de DTR afin d'augmenter l'affinité de la protéine pour HB-EGF. Ces mutations étaient destinées à augmenter l'enthalpie de l'interaction en favorisant la formation de liaisons hydrogènes, de ponts salins et/ou de contacts de Van der Wals entre les deux protéines. L'effet potentiel des mutations sélectionnées sur la structure de la protéine est indiqué dans le Tableau VI.

**Tableau VI : Remarques d'ordre structural sur les résidus sélectionnés pour augmenter l'affinité de la protéine DTR et effet escompté des mutations**

| **Positions** | **Mutations** | **Remarques d'ordre structural et interactions potentielles** |
|---|---|---|
| F389 | - | Dans un brin béta, en bordure de la région d'interaction avec HB-EGF, para position proche d'une molécule d'eau de l'interface structurée et de HB-EGF H139 |
| | F389Y | Donneur ou accepteur de liaison hydrogène pour une molécule d'eau de l'interface structurée ou HB-EGF H139 |
| H391 | - | Dans un brin béta, en bordure de la région d'interaction avec HB-EGF, donneur de liaison hydrogène pour HB-EGF E141 |
| | H391K | Liaison ionique avec HB-EGF E141 |
| G510 | - | Dans un brin béta, au centre de la région interaction avec HB-EGF, à côté d'une cavité entre DTR et HB-EGF, face au groupe CO du squelette de HB-EGF C132 |
| | G510A | Changement le plus conservatif pour remplir la cavité et augmenter les contacts de Van der Waals |
| | G510M | Chaîne latérale hydrophobe flexible pour remplir la cavité et augmenter les contacts de Van der Waals |
| | G510Q | Donneur de liaison hydrogène pour le groupe CO du squelette de HB-EGF C132 et/ou accepteur de liaison hydrogène H pour le groupe NH du squelette de HB-EGF C134. |
| | G510S | Petite chaîne latérale polaire pour remplir la cavité et augmenter les contacts de Van der Waals |
| T521 | - | Dans un pseudo coude de type II, en bordure de la région d'interaction avec HB-EGF |
| | T521R | Donneur de liaison hydrogène pour le groupe CO du squelette de HB-EGF K111 et/ou le groupe CO du squelette de HB-EGF K113 |
| Q515 | - | Dans un brin béta, en bordure de la région d'interaction avec HB-EGF, donneur de liaison hydrogène pour le groupe CO du squelette de HB-EGF L127 |
| | Q515E | Dans un réseau de liaison ionique et hydrogène impliquant K522R, HB-EGF R128, le groupe CO du squelette de HB-EGF R128, le groupe CO du squelette de HB-EGF L127 |
| K522 | - | Dans un brin béta, en bordure de la région d'interaction avec HB-EGF, donneur de liaison hydrogène T517 |
| | K522R | Dans un réseau de liaison ionique et hydrogène impliquant Q515E, HB-EGF R128, le groupe CO du squelette de HB-EGF R128, le groupe CO du squelette de HB-EGF L127 |

Les mutations ont été introduites dans la protéine DTR1 par mutagenèse dirigée (Tableau VII).

**Tableau VII : Mutations pour améliorer l'affinité de liaison de DTR à l'HB-EGF identifiées par modélisation moléculaire et testées expérimentalement**

| | |
|---|---|
| F389Y | G510Q |
| H391K | G510S |
| F389Y/ H391K | T521R |
| G510A | Q515E/K522R |
| G510M | F389Y/H391K/Q515E/K522R |

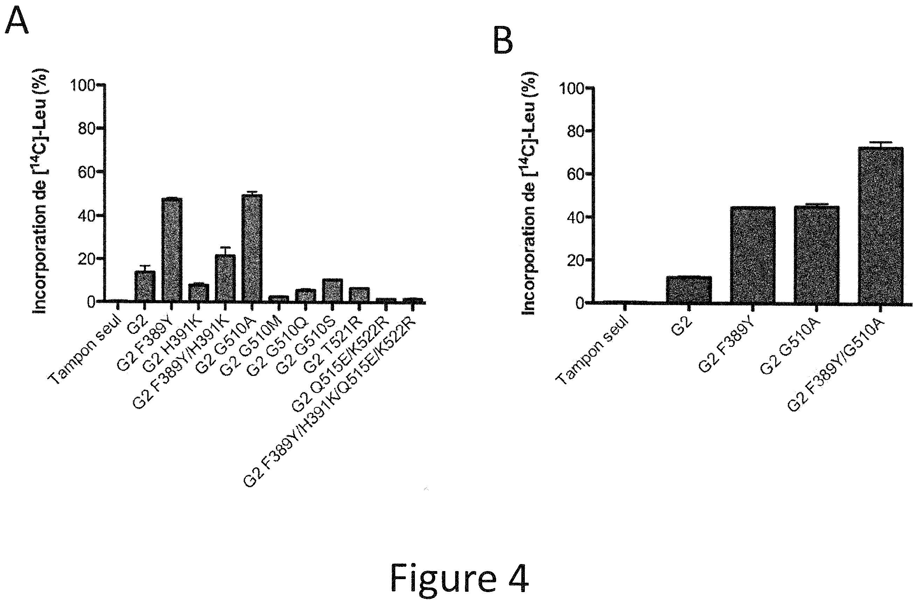

Les protéines ont été exprimées chez *E. coli.* Elles ont été testées pour leur capacité à inhiber la liaison de la toxine diphtérique au pro-HB-EGF selon le test de cytotoxicité décrit à l'exemple 1. La Figure 4A montre la capacité de chaque mutant à inhiber ou non la toxicité de la toxine diphtérique sur des cellules Vero. Les résultats montrent que seuls les mutants F389Y et G510A inhibent de façon importante la toxicité de la toxine diphtérique. L'introduction de ces deux mutations dans DTR1 entraine un effet cumulatif (Figure 4B).

Le mutant le plus actif, correspondant à la protéine DTR1 portant les mutations F389Y et G510A a été appelé DTR3 (SEQ ID NO : 8). Son affinité de liaison à l'HB-EGF a été évaluée par la capacité de doses croissantes de DTR3 à inhiber l'effet toxique de doses croissantes de toxine diphtérique sur des cellules Vero comme décrit à l'exemple 2. Le Kd estimé par régression de Schild à partir des EC50 des courbes d'inhibition est présenté au Tableau VIII.

**Tableau VIII : Affinité pour HB-EGF**

| **Protéine** | **Kd (pM)** |
|---|---|
| DTR3 | ∼ 9,5 |

Cette valeur suggère que DTR3 est au moins 300 fois plus affine que CRM197 pour HB-EGF et 5 fois plus affine que DTR1.

### Exemple 4 : Diminution de l'imunogénicité de la protéine DTR par suppression des principaux épitopes T CD4

La capacité de 25 peptides chevauchants couvrant la totalité de la séquence de DTR_{WT} à activer des lymphocytes T CD4 spécifiques a été testée par ELISPOT, dans des expériences d'immunisation *in vitro* à partir de lymphocytes T CD4 et de cellules dendritiques purifiés du sang de 7 donneurs sains, d'âge et de phénotype HLA-DRB1 différents.

Les résultats montrent que la réponse immunitaire contre la protéine DTR_{WT} est dirigée majoritairement contre cinq régions épitopiques recouvrant 60% de la protéine et contre lesquelles au moins 71% des donneurs ont répondu, soit 5 donneurs sur 7 étudiés (Tableau IX):
- la région L₄₂₇-L₄₄₁ (Peptide 9) contre laquelle des lymphocytes T CD4 spécifiques ont été détectés pour la totalité des donneurs étudiés avec une amplitude élevée (51 lignées parmi 230 lignées criblées soit 22%),
- les régions H₃₉₁-Q₄₁₁ / S₄₅₁-D₄₆₅ / S₄₇₅-N₅₀₂ (Peptides 3-4, 13 et 17-18-19 respectivement) contre lesquelles globalement des cellules T CD4 spécifiques ont été détectées pour 86% des donneurs (soit 6 donneurs/7) avec une amplitude un peu plus modérée que pour la région décrite ci-dessus (20 à 30 lignées lymphocytaires T CD4 spécifiques soit 8,5 à 13%), et
- la région S₅₀₆-H₅₂₀ (peptide 22) contre laquelle des cellules T CD4 spécifiques ont été détectées pour 71% des donneurs avec une amplitude de 8%.

**Tableau IX : Bilan, par peptide et par donneur, des lignées lymphocytaires T CD4 spécifiques de la protéine DTR**

| | **Donneurs** | **P668** | **P661** | **P659** | **P663** | **P664** | **P667** | **P658** | **Nbre* total lignées T CD4** | **Fréquence de répondeurs** |
|---|---|---|---|---|---|---|---|---|---|---|
| | **HLA-DRB1** | DR3 | DR1 | DR13 | DR4 | DR1 | DR8 | DR3 | | |
| | | DR13 | DR11 | DR7 | DR11 | DR4 | DR15 | DR7 | | |
| **Petides/Age** | | 52 | 44 | 29 | 62 | 57 | 44 | 30 | | |
| **1** | **378-392** | | | **1** | | | | | 1 | 14 |
| **2** | **385-399** | | 1 | | **4** | | 2 | | 7 | 43 |
| **3** | **391-405** | 1 | | 3 | 2 | 1 | | **4** | **11** | **71** |
| **4** | **397-411** | 1 | | 3 | 1 | 1 | **13** | **8** | **27** | **86** |
| **5** | **403-417** | | | | | 1 | | | 1 | 14 |
| **6** | **409-423** | | 1 | | | **6** | | | 7 | 29 |
| **7** | **415-429** | | | | | | | 1 | 1 | 14 |
| **8** | **421-435** | | 1 | | | | 1 | | 2 | 29 |
| **9** | **427-441** | 3 | 3 | **5** | **3** | **13** | 4 | **20** | **51** | **100** |
| **10** | **433-447** | 1 | | 2 | | 1 | | **18** | 22 | 57 |
| **11** | **439-453** | 2 | 2 | | | | | 1 | 5 | 43 |
| **12** | **445-459** | **9** | | **5** | | | | 3 | 17 | 43 |
| **13** | **451-465** | **9** | | **14** | 1 | 2 | 3 | 1 | **30** | **86** |
| **14** | **457-471** | | | 2 | 1 | | | 1 | 4 | 43 |
| **15** | **463-477** | | | | | | | **10** | 10 | 14 |
| **16** | **469-483** | 2 | | **1** | | | | | 3 | 29 |
| **17** | **475-489** | **7** | 2 | **8** | **5** | | | 1 | **21** | **71** |
| **18-19** | **482-502** | **4** | **1** | **5** | **4** | | **1** | **3** | **18** | **86** |
| **20** | **494-508** | | | 2 | | | | 2 | 4 | 29 |
| **21** | **500-514** | | | **1** | 1 | | | **4** | 6 | 43 |
| **22** | **506-520** | **1** | | 2 | **6** | 4 | **5** | | **18** | **71** |
| **23** | **512-526** | | | | 3 | 2 | | | 5 | 29 |
| **24** | **518-532** | | | 2 | | | **1** | | 3 | 29 |
| **25** | **521-535** | | | 1 | | | | | 1 | 14 |
| | **Total** | 27 | 16 | **44** | 23 | 28 | 30 | **40** | | |
| | **%** | 30 | 13 | **49** | 19 | 31 | 33 | **44** | | |

| | | | | | | | | | | |
|---|---|---|---|---|---|---|---|---|---|---|
| *Nombre total de lignées lymphocytaires T CD4 spécifiques d'un peptide parmi les 230 lignées criblées | | | | | | | | | | |

5 régions épitopiques étant identifiées, il est possible d'envisager la mutation de ces épitopes afin de réduire leur liaison aux molécules HLA-II. Le serveur ProPred (http://www.imtech.res.in/raghava/propred/) a été utilisé pour prédire au sein de ces cinq régions de DTR_{WT} les séquences capables de lier les 8 allèles HLA de classe II les plus communs dans la population (Tableau X). La même analyse appliquée aux séquences mutées de DTR1 montre que les mutations introduites pour améliorer la solubilité de DTR diminuent l'immunogénicité de la protéine (épitopes prédits pour la région 378-403). En effet, l'épitope prédit pour lier DRB1_0301 disparaît et un des épitopes prédit pour lier DRB1_0401 voit son seuil augmenter, soit son affinité prédite pour la molécule HLA diminuer.

**Tableau X. Prédiction des séquences capables de lier les 8 allèles HLA de classe II les plus communs (DRB1_0101, DRB1_0301, DRB1_0401, DRB1_0701, DRB1_1101, DRB1_1301, DRB1_1501, DRB5_0101) au sein des 5 régions identifiées comme immunogènes par test d'activation de lymphocytes T CD4. Les séquences peptidiques correspondent aux séquences SEQ ID NO : 43 à 109. La deuxième colonne du tableau donne la région étudiée. La première colonne indique les mutations proposées pour inhiber la liaison des séquences prédites aux molécules HLA de classe II. Les colonnes suivantes indiquent pour chaque allèle HLA considéré la présence ou non de séquence capable de lier cette molécule HLA. Chaque séquence est précédée par une valeur de seuil reflétant l'intensité de liaison : 1/2 (liaison forte), 3/4 (liaison moyenne), 5/6 (liaison faible). Les résidus en gras correspondent aux mutations proposées pour abolir la liaison des séquences à l'allèle HLA considéré.**

| **Variant** | **Sequence (378-403)** | **DRB1_0101** | **DRB1_0301** | **DRB1_0401** | **DRB1_0701** | **DRB1_1101** | **DRB1_1301** | **DRB1_1501** | **DRB5_0101** |
|---|---|---|---|---|---|---|---|---|---|
| WT | MGYSPGHKTQPFLHDGYAVSWNTVED | - | 6:FLHDGYAVS | 3:FLHDGYAVS | - | - | - | - | - |
| | | | | 5:YAVSWNTVE | | | | | |
| DTR1 | MG**K**SPGHKT**E**PFTHDGY**T**VSWNTVED | - | - | 5:F**T**HDGYTVS | - | - | - | - | - |
| | | | | 5:Y**T**VSWNTVE | | | | | |

| **Variant** | **Sequence (391-411)** | **DRB1_0101** | **DRB1_0301** | **DRB1_0401** | **DRB1_0701** | **DRB1_1101** | **DRB1_1301** | **DRB1_1501** | **DRB5_0101** |
|---|---|---|---|---|---|---|---|---|---|
| DTR1 | HDGYTVSWNTVEDSIIRTGFQ | - | - | 4:VSWNTVEDS | 6:WNTVEDSII | - | - | - | - |
| | | | | 5:YTVSWNTVE | | | | | |
| N399K | HDGYTVSW**K**TVEDSIIRTGFQ | - | - | - | 5:WKTVEDSII | - | 6:VSW**K**TVEDS | - | - |
| V401Q | HDGYTVSWNT**Q**EDSIIRTGFQ | - | - | 5:YTVSWNT**Q**E | - | - | - | - | - |
| N399K | HDGYTVSWKT**Q**EDSIIRTGFQ | - | - | - | - | - | - | - | - |
| V401Q | | | | | | | | | |
| **Variant** | **Sequence (427-441)** | **DRB1_0101** | **DRB1_0301** | **DRB1_0401** | **DRB1_0701** | **DRB1_1101** | **DRB1_1301** | **DRB1_1501** | **DRB5_0101** |
| DTR1 | LPIAGVLLPTIPGKL | - | 6:VLLPTIPGK | - | 3:LLPTIPGKL | 3:LPIAGVLLP | 4:LPIAGVLLP | - | 2:LLPTIPGKL |
| L427Q | **Q**PIAGVLLPTIPGKL | - | 6:VLLPTIPGK | - | 3:LLPTIPGKL | - | - | - | 2:LLPTIPGKL |
| T436K | LPIAGVLLP**K**IPGKL | - | 6:VLLP**K**IPGK | - | - | 3:LPIAGVLLP | 4:LPIAGVLLP | - | - |
| L427Q | **Q**PIAGVLLP**K**IPGKL | - | 6:VLLP**K**IPGK | - | - | - | - | - | - |
| T436K | | | | | | | | | |

| **Variant** | **Sequence (451-465)** | **DRB1_0101** | **DRB1_0301** | **DRB1_0401** | **DRB1_0701** | **DRB1_1101** | **DRB1_1301** | **DRB1_1501** | **DRB5_0101** |
|---|---|---|---|---|---|---|---|---|---|
| DTR1 | SVNGRKIRMRCRAID | - | 3:IRMRCRAID | - | - | 3:IRMRCRAID | 1:VNGRKIRMR | 4:IRMRCRAID | - |
| | | | | | | | 1:IRMRCRAID | | |
| I457D | SVNGRK**D**RMRCRAID | - | - | - | - | - | - | - | - |
| I457E | SVNGRK**E**RMRCRAID | - | - | - | - | - | - | - | - |
| V452T | S**T**NGRKIRMRCRAID | - | 3:IRMRCRAID | - | - | 3:IRMRCRAID | 1:IRMRCRAID | 4:IRMRCRAID | - |
| R460T | SVNGRKIRM**T**CRAID | - | 3:IRM**T**CRAID | - | - | 3:IRMTCRAID | 3:VNGRKIRM**T** | 6:IRM**T**CRAID | - |
| | | | | | | | 3:IRM**T**CRAID | | |
| A463D | SVNGRKIRMRCR**D**ID | - | 5:IRMRCR**D**ID | - | - | - | 1:VNGRKIRMR | - | - |
| | | | | | | | 3:IRMRCR**D**ID | | |
| V452T | STNGRKIRM**T**CR**D**ID | - | 5:IRM**T**CR**D**ID | - | - | - | - | - | - |
| R460T | | | | | | | | | |
| A463D | | | | | | | | | |

| **Variant** | **Sequence (475-502)** | **DRB1_0101** | **DRB1_0301** | **DRB1_0401** | **DRB1_0701** | **DRB1_1101** | **DRB1_1301** | **DRB1_1501** | **DRB5_0101** |
|---|---|---|---|---|---|---|---|---|---|
| DTR1 | SPVYVGNGVHANLHVAFHRSSSEKIHSN | 3:YVGNGVHAN | - | 1:YVGNGVHAN | 1:FHRSSSEKI | 2:YVGNGVHAN | 1:LHVAFHRSS | - | - |
| | | 3:FHRSSSEKI | | 1:FHRSSSEKI | | 5:LHVAFHRSS | 4:YVGNGVHAN | | |
| | | 4:VYVGNGVHA | | | | | | | |
| Y478T | SPVTVGNGVHANLHVAFHRSSSEKIHSN | 3:FHRSSSEKI | - | 1:FHRSSSEKI | 1:FHRSSSEKI | 5:LHVAFHRSS | 1:LHVAFHRSS | - | - |
| V483D | SPVYVGNG**D**HANLHVAFHRSSSEKIHSN | 3:FHRSSSEKI | - | 1:FHRSSSEKI | 1:FHRSSSEKI | 5:LHVAFHRSS | 1:LHVAFHRSS | - | - |
| | | | | 5:YVGNG**D**HAN | | | | | |
| V483E | SPVYVGNG**E**HANLHVAFHRSSSEKIHSN | 3:FHRSSSEKI | - | 1:FHRSSSEKI | 1:FHRSSSEKI | 5:LHVAFHRSS | 1:LHVAFHRSS | - | - |
| V483H | SPVYVGNG**H**HANLHVAFHRSSSEKIHSN | 3:FHRSSSEKI | - | 1:FHRSSSEKI | 1:FHRSSSEKI | 5:VYVGNGH**H**A | 1:LHVAFHRSS | 6:VYVGNG**H**HA | - |
| | | 5:VYVGNG**H**HA | | | | 5:LHVAFHRSS | | | |
| V483Q | SPVYVGNG**Q**HANLHVAFHRSSSEKIHSN | 3:FHRSSSEKI | - | 1:FHRSSSEKI | 1:FHRSSSEKI | S:YVGNG**Q**HAN | 1:LHVAFHRSS | - | - |
| | | S:VYVGNG**Q**HA | | | | 5:LHVAFHRSS | | | |
| | | 6:YVGNG**Q**HAN | | | | | | | |
| A490G | SPVYVGNGVHANLHV**G**FHRSSSEKIHSN | 3:YVGNGVHAN | - | 1:YVGNGVHAN | 1:F**H**RSSSEKI | 2:YVGNGVHAN | 4:YVGNGVHAN | - | - |
| | | 3:FHRSSSEKI | | 1:FHRSSSEKI | | | S:LHV**G**FHRSS | | |
| | | 4:VYVGNGVHA | | 6:V**G**FHRSSSE | | | | | |
| H492E | SPVYVGNGVHANLHVAF**E**RSSSEKIHSN | 3:YVGNGVHAN | - | 1:YVGNGVHAN | 1:F**E**RSSSEKI | 2:YVGNGVHAN | 4:YVGNGVHAN | - | - |
| | | 4:VYVGNGVHA | | 2:F**E**RSSSEKI | | | S:LHVAF**E**RSS | | |
| | | 5:F**E**RSSSEKI | | | | | | | |
| S494K | SPVYVGNGVHANLHVAFHR**K**SSEKIHSN | 3:YVGNGVHAN | - | 1:YVGNGVHAN | 2:FHR**K**SSEKI | 2:YVGNGVHAN | 1:LHVAFHR**K**S | - | - |
| | | 4:VYVGNGVHA | | S:FHR**K**SSEKI | | 5:LHVAFHR**K**S | 4:YVGNGVHAN | | |
| | | | | | | | 4:VAFHR**K**SSE | | |
| S496K | SPVYVGNGVHANLHVAFHRSS**K**EKIHSN | 3:YVGNGVHAN | 4:FHRSSKE**K**I | 1:YVGNGVHAN | 1:FHRSS**K**EKI | 2:YVGNGVHAN | 1:LHVAFHRSS | - | - |
| | | 4:VYVGNGVHA | | | | 5:LHVAFHRSS | 4:YVGNGVHAN | | |
| | | | | | | S:FHRSS**K**EKI | | | |
| Y478T | SPV**T**VGNGVHANLHV**G**F**E**R**K**S**K**EKIHSN | - | - | - | - | - | - | - | - |
| A490G | | | | | | | | | |
| H492E | | | | | | | | | |
| S494K | | | | | | | | | |
| S496K | | | | | | | | | |

| **Variant** | **Sequence (506-520)** | **DRB1_0101** | **DRB1_0301** | **DRB1_0401** | **DRB1_0701** | **DRB1_1101** | **DRB1_1301** | **DRB1_1501** | **DRB5_0101** |
|---|---|---|---|---|---|---|---|---|---|
| DTR1 | SDSIGVLGYQKTVDH | - | - | 1:LGYQKTVDH | - | 6:LGYQKTVDH | S:LGYQKTVDH | - | - |
| T517D | SDSIGVLGYQK**D**VDH | - | - | - | - | - | - | - | - |
| T517E | SDSIGVLGYQK**E**VDH | - | - | - | - | - | - | - | - |

La prédiction des résidus d'ancrage des séquences prédites pour lier les molécules HLA de classe II a permis de proposer une série de mutations indiquées en gras dans le Tableau X, destinées à supprimer des épitopes T potentiels. Les mutations ont été choisies de manière à éviter une déstabilisation potentielle de la protéine, ce qui a été testé *in silico* par modélisation moléculaire. Toute mutation affectant le site de liaison à l'HB-EGF a été exclue. L'effet potentiel des mutations proposées sur la structure de la protéine est indiqué dans le Tableau XI.

**Tableau XI : Remarques d'ordre structural sur les résidus sélectionnés pour supprimer des épitopes T CD4 de la protéine DTR et effet escompté des mutations**

| **Positions** | **Mutations** | **Remarques d'ordre structural et interactions potentielles** |
|---|---|---|
| N399 | - | Exposé au solvant, dans une boucle, pas de liaison hydrogène stable |
| | N399K | Liaison ionique avec D417 et donneur de liaison hydrogène pour le groupe CO du squelette de N486. |
| V401 | - | Exposé au solvant, dans un coude de type I |
| | V401Q | Accepteur de liaison hydrogène pour K385 |
| L427 | - | Partiellement enfoui, dans un brin béta, contact de Van der Waals contacts avec Y394 |
| | L427Q | donneur de liaison hydrogène pour le groupe CO du squelette de D392, chaîne latérale hydrocarbonée établissant des contacts de Van der Waals avec Y394 |
| T436 | - | Partiellement enfoui, dans une boucle, donneur de liaison hydrogène pour le groupe CO du squelette de G466, contacts de Van der Waals avec V443 |
| | T436H | Accepteur de liaison hydrogène pour T469, contacts de Van der Waals avec V443 |
| | T436K | Liaison ionique avec A463D |
| I457 | - | En grande partie enfoui; dans un brin béta ; contacts de Van der Waals avec I450, V452, P473, V477 |
| | I457D | Accepteur de liaison hydrogène pour S475, le groupe NH du squelette de S475, le groupe NH du squelette de K474 |
| | I457E | Accepteur de liaison hydrogène pour S475, le groupe NH du squelette de S475, le groupe NH du squelette de K474, ou le groupe NH du squelette de V452 |
| V452 | - | Partiellement enfoui, dans un brin beta, contacts de Van der Waals d'un groupe méthyle de la chaîne latérale avec I450, I457, V477 |
| | V452T | contacts de Van der Waals du group méthyle de la chaîne latérale avec I450, I457, V477 |
| A463 | - | Exposé au solvant, dans un brin béta |
| | A463D | Liaison ionique avec T436K |
| R460 | - | Exposé au solvant, dans un brin béta, fait partie d'un patch |
| | | basique contenant d'autres arginines interagissant potentiellement avec des groupes héparane sulfate liés à la membrane plasmique, proche de HB-EGF, donneur de liaison hydrogène pour le groupe CO du squelette de P473 |
| | R460T | Résidu béta branché |
| Y478 | - | Exposé au solvant, dans un brin béta, contacts de Van der Waals avec P426, P428, P476 |
| | Y478T | Résidu béta branché |
| V483 | - | Exposé au solvant, dans une boucle |
| | V483D | |
| | V483E | accepteur de liaison hydrogène pour V452T, N453 |
| | V483H | donneur de liaison hydrogène pour le groupe CO du squelette de Y478 |
| | V483Q | accepteur ou donneur de liaison hydrogène pour V452T, donneur de liaison hydrogène pour le groupe CO du squelette de Y478 |
| A490 | - | Partiellement enfoui, dans un brin béta |
| | A490G | |
| H492 | - | Exposé au solvant, dans un brin béta, accepteur ou donneur de liaison hydrogène pour S494 |
| | H492E | Liaison ionique avec S494K |
| S494 | - | Exposé au solvant, dans une boucle, accepteur de liaison hydrogène pour H492 |
| | S494K | Liaison ionique avec H492E |
| S496 | - | Exposé au solvant, dans une boucle |
| | S496K | Donneur de liaison hydrogène pour Q411 |
| T517 | - | Exposé au solvant, dans un brin béta, accepteur de liaison hydrogène pour K522 |
| | T517D | Liaison ionique avec K522 |
| | T517E | Liaison ionique avec K522 |

Les mutations indiquées dans le Tableau XII ont été introduites individuellement ou parfois en combinaison dans la séquence de la protéine DTR3, en fonction des données de modélisation, puis cumulées progressivement lorsqu'elles n'altéraient ni la production de la protéine recombinante, ni son activité biologique. L'activité biologique des mutants a été testée par inhibition de la toxicité de la toxine diphtérique sur cellules Vero.

**Tableau XII : Mutations introduites dans DTR3 pour supprimer des épitopes T CD4.**

| **N399K** | A463S | **H492E** |
|---|---|---|
| V401Q | A463E | **S494K** |
| L427Q | A463D | S496K |
| L427N | A463G | **H492E/S494K** |
| L427S | T436K/A463D | H492E/S496K |
| T436K | V452T/R460T/A463D | S494K/S496K |
| **T436H** | Y478T | H492E/S494K/S496K |
| **V452T** | V483D | **E497D** |
| I457D | V483E | T436H/E497D |
| I457E | V483H | T517D |
| R460T | **V483Q** | **T517E** |
| A463T | A490G | |

| | | |
|---|---|---|
| * En gras, les mutations n'affectant pas de manière importante l'expression et l'activité de la protéine, qui ont donc été retenues. | | |

Ces expériences ont permis de retenir successivement les mutations suivantes : N399K, V452T, T517E, V483Q, H492E, S494K, T436H et E497D

La protéine dérivée de DTR3 et cumulant les mutations N399K, V452T, T517E, V483Q, H492E et S494K est appelée DTR8 (SEQ ID NO : 9) ; elle a un PM de 17458 Da. Ces mutations ont permis de supprimer 10 des 26 épitopes T CD4 identifiés dans DTR_{WT} (Tableau XIII).

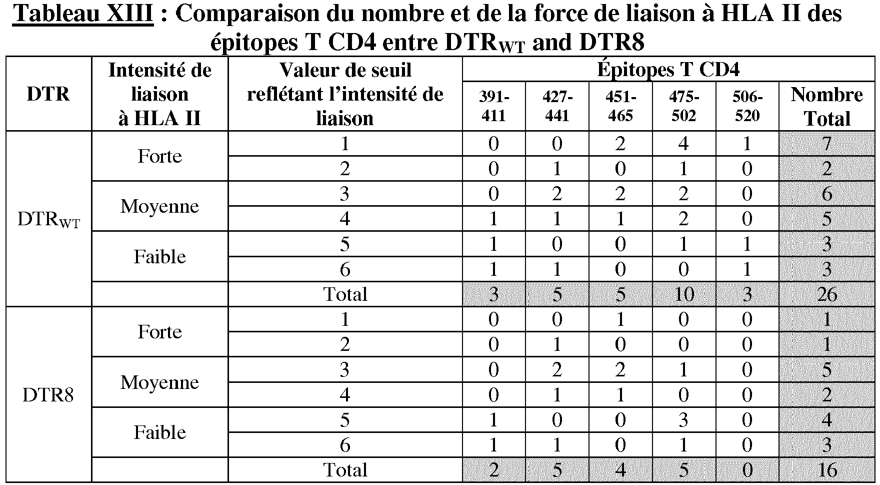

Parmi ces 10 épitopes se trouvent 7 des 9 épitopes prédis comme immunodominants de DTR_{WT}. La capacité de la protéine résultante, DTR8, d'induire une réponse immunitaire de type CD4, c'est-à-dire productrice d'anticorps, se trouve ainsi considérablement diminuée par rapport à celle de DTR_{WT} dans sa forme sauvage.

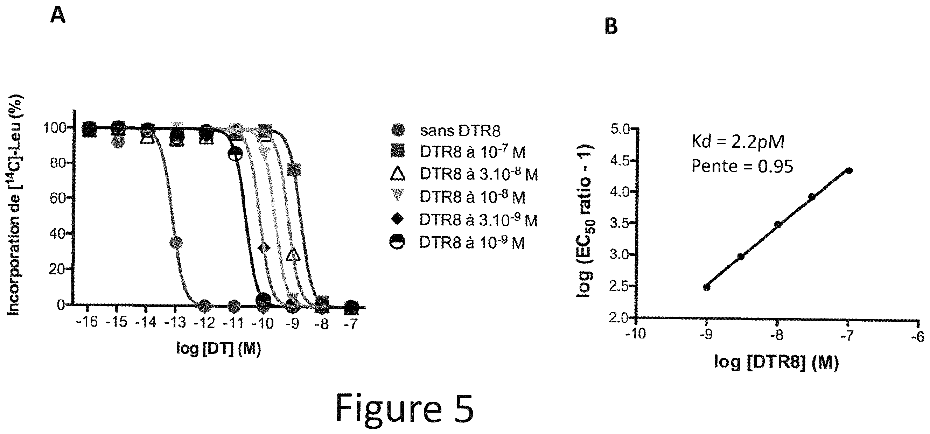

DTR8 contient six mutations de plus que DTR3. Toute mutation et combinaison de mutations étant susceptible d'altérer la fonction d'une protéine, il est nécessaire d'évaluer l'effet de ces mutations supplémentaires sur l'affinité de liaison de DTR pour HB-EGF. L'activité de liaison de la protéine DTR8 à HB-EGF a été évaluée par sa capacité à inhiber l'intoxication de cellules Vero par la toxine diphtérique, et donc la liaison de la toxine au pro-HB-EGF. Les résultats (Figure 5) montrent que DTR8 inhibe l'intoxication de cellules Vero par la toxine diphtérique de manière dose-dépendante. L'effet inhibiteur de DTR8 a été comparé à celui de CRM197, DTR_{WT} solubilisé dans 0,5% de sarkosyl, DTR3 et DTR1 (Figure 3). Les Kd estimés par régression de Schild pour les quatre interactions donnent les valeurs suivantes (Tableau XIV).

**Tableau XIV : Affinité des protéines pour l'HB-EGF**

| **Protéine** | **Kd (pM)** |
|---|---|
| DTR8 | 2,2 |
| DTR3 | 9,5 |
| DTR1 | 49 |
| DTR_{WT} (+ 0,5% sarkosyl) | 6500 |
| CRM197 | 3100 |

De façon remarquable et inattendue, l'ajout des six mutations destinées à réduire l'immunogénicité de DTR a contribué à augmenter son affinité pour HB-EGF. Au total, DTR8 est environ 1400 fois plus affine pour HB-EGF que CRM197.

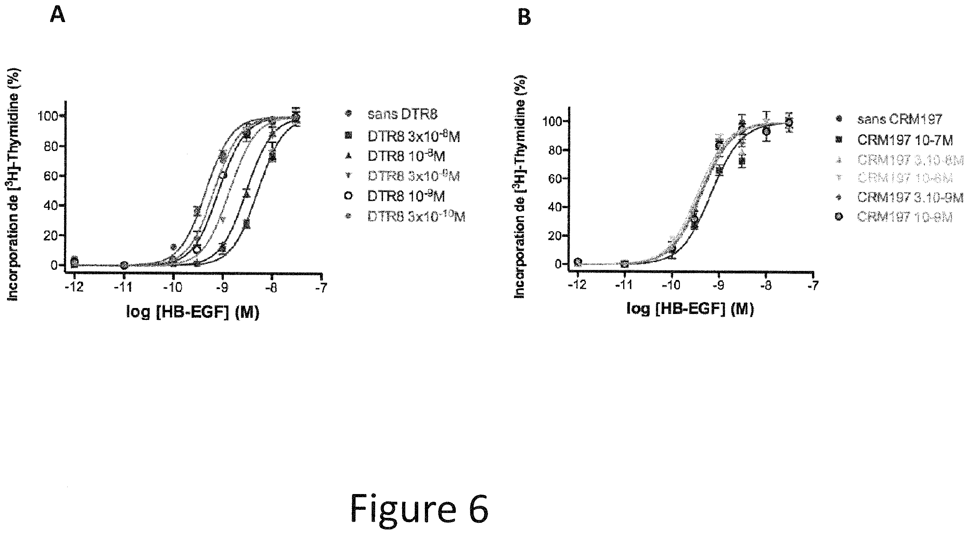

L'activité de liaison de la protéine DTR8 à HB-EGF a été évaluée également par sa capacité à inhiber la liaison de l'HB-EGF à l'EGFR chez une lignée cellulaire Ba/F3 transfectées par le gène de l'EGFR et dépendante de l'HB-EGF pour sa croissance. Les résultats (Figure 6) montrent que DTR8 inhibe la prolifération des cellules dépendantes de l'HB-EGF de manière dose-dépendante. L'effet inhibiteur de DTR8 a été comparé à celui de CRM197 (Figure 6). Les résultats montrent qu'il faut au moins 300 fois plus de CRM197 pour obtenir un effet inhibiteur équivalent. En conséquence, DTR8 est au moins 300 fois plus efficace que CRM197 pour se lier à l'HB-EGF en solution.

En conclusion, ces résultats montrent que la protéine DTR8 est capable de se lier aux molécules pro-HB-EGF à la surface des cellules (Figure 5) et à l'HB-EGF en solution (Figure 6) et de bloquer leur activité biologique. L'estimation des Kd des interactions suggère que DTR8 est 1400 fois plus puissant que CRM197 pour se lier à l'HB-EGF.

### Exemple 5 : Evaluation de l'antigénicité des protéines DTR1 et DTR8

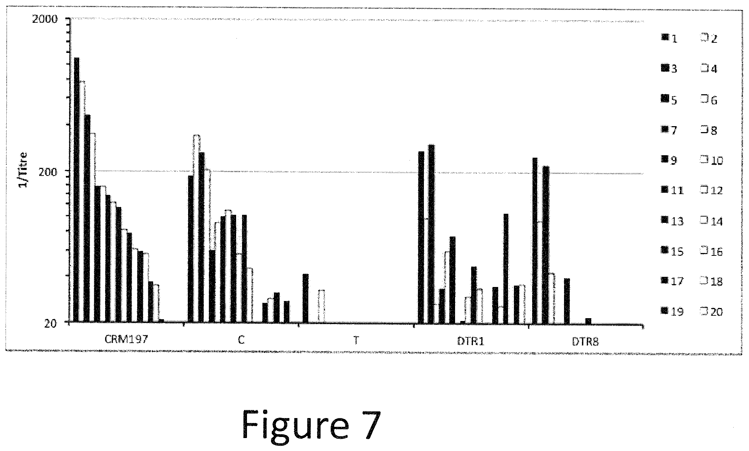

La population occidentale est vaccinée contre la toxine diphtérique. Les individus susceptibles de bénéficier d'un traitement par une protéine thérapeutique de type DTR8 pourraient donc présenter des anticorps capables de réagir contre celle-ci. La capacité des sérums de 20 donneurs sains à reconnaître la toxine diphtérique (sous sa forme mutée CRM197), ces différents domaines (catalytique (C), de translocation (T) et DTR (sous la forme mutée, soluble DTR1)) a été comparé avec la protéine DTR8, par ELISA (Figure 7). Un titre d'anticorps ≤ 20 correspond au bruit de fond du test et donc à une absence de reconnaissance. Un seuil du titre à 30 a été fixé arbitrairement pour distinguer les sérums présentant une réponse faible ou forte.

Les résultats (Figure 7) montrent que 16/20 donneurs possèdent des anticorps contre la toxine diphtérique. 14/20 donneurs présentent une réponse anticorps moyenne à forte. Cependant, la majorité des anticorps présents dans chaque sérum considéré individuellement sont dirigés contre le domaine C de la toxine (Figure 7). En effet, 12 sérums présentent une réponse supérieure au seuil (réponse moyenne à forte) et 4 sérums présentent une réponse inférieure au seuil (réponse faible). Si l'on considère la réactivité des sérums contre la forme soluble du domaine R (DTR1), 15/20 sérums reconnaissent le domaine R. Cependant, seuls 7 sérums présentent une réponse au dessus du seuil (réponse moyenne à forte). De façon remarquable, la protéine DTR8 est considérablement moins bien reconnue par les sérums des donneurs que DTR1. En effet, seulement 3/20 sérums présentent une réactivité moyenne à forte contre DTR8 et 2 sérums présentent une réactivité faible (Figure 7).

En conclusion, ces résultats montrent que l'antigénicité de la protéine DTR8 est faible et considérablement réduite par rapport à celle de CRM197. Cette antigénicité est réduite aussi par rapport à celle de la protéine DTR1, correspondant à la forme soluble de DTR portant le plus faible nombre de mutations. En d'autres termes, les mutations introduites dans DTR pour augmenter son affinité pour HB-EGF et diminuer son immunogénicité contribuent à réduire considérablement son antigénicité.

## Revendications

1. Protéine recombinante, comprenant un domaine R isolé de la toxine diphtérique comprenant une séquence d'acides aminés ayant au moins 70 % d'identité avec les résidus 380 à 535 de la séquence d'acides aminés SEQ ID NO : 1, ladite séquence comprenant la combinaison de substitutions suivante :
- Y380K et L390T,
et ladite séquence étant dépourvue à l'extrémité N- ou C-terminale dudit domaine R, de la séquence du domaine C et du domaine T de la toxine diphtérique et de la séquence d'une protéine ou d'un domaine de protéine capable d'améliorer la stabilité ou la purification dudit domaine R, et ladite protéine recombinante étant un ligand inhibiteur de l'HB-EGF.

2. Protéine selon la revendication 1, comprenant au moins les substitutions Y380K, Q387E et L390T.

3. Protéine selon la revendication 1 ou 2, comprenant de plus la substitution A395T.

4. Protéine selon l'une quelconque des revendications 1 à 3, comprenant également au moins l'une des substitutions F389Y et/ou G510A.

5. Protéine selon l'une quelconque des revendications 1 à 4, comprenant également au moins une substitution sélectionnée dans le groupe constitué par : N399K, V452T, T517E, V483Q, H492E, S494K, T436H et E497D.

6. Protéine selon la revendication 5, comprenant les substitutions N399K, V452T, T517E, V483Q, H492E et S494K.

7. Protéine selon l'une quelconque des revendications 1 à 6, comprenant ou consistant en l'une des séquences d'acides aminés SEQ ID NO : 2 à 5 et SEQ ID NO : 7 à 9.

8. Protéine selon l'une quelconque des revendications 1 à 7, marquée par un traceur détectable.

9. Polynucléotide isolé codant pour une protéine recombinante selon l'une quelconque des revendications 1 à 8.

10. Vecteur recombinant de clonage et/ou d'expression comprenant un polynucléotide selon la revendication 9.

11. Cellule modifiée par un vecteur selon la revendication 10.

12. Composition pharmaceutique, comprenant au moins une protéine recombinante selon l'une quelconque des revendications 1 à 7 ou un vecteur recombinant d'expression selon la revendication 10, et un véhicule pharmaceutiquement acceptable.

13. Protéine recombinante selon l'une quelconque des revendications 1 à 7 ou vecteur recombinant d'expression selon la revendication 10, pour utilisation dans le traitement de maladies associées à une activation de la voie HB-EGF/EGFR sélectionnées dans le groupe constitué par : les glomérulonéphrites rapidement progressives, les cancers, le vasospasme associé aux contusions cérébrales, l'hypertrophie cardiaque, l'hyperplasie des cellules musculaires lisses, l'hypertension pulmonaire, les rétinopathies diabétiques et la resténose artérielle.

14. Utilisation d'une protéine marquée selon la revendication 8 pour le diagnostic *in vitro* d'une maladie liée à une activation de la voie de l'HB-EGF/EGFR sélectionnée dans le groupe constitué par : les glomérulonéphrites rapidement progressives, les cancers, le vasospasme associé aux contusions cérébrales, l'hypertrophie cardiaque, l'hyperplasie des cellules musculaires lisses, l'hypertension pulmonaire, les rétinopathies diabétiques et la resténose artérielle.

## Patentansprüche

1. Rekombinantes Protein, welches eine isolierte R-Domäne des Diphterietoxins umfasst, welche eine Aminosäuresequenz mit wenigstens 70% Identität mit den Resten 380 bis 535 der Aminosäuresequenz SEQ ID NR. 1 aufweist, wobei die Sequenz die folgende Substitutionenkombination aufweist:
- Y380K und L390T,
und wobei die Sequenz am N-terminalen oder C-terminalen Ende der R-Domäne frei von der Sequenz der C-Domäne und der T-Domäne des Diphterietoxins ist, sowie von der Sequenz eines Proteins oder einer Proteindomäne, die die Stabilität oder die Reinigung der R-Domäne verbessern kann, und wobei das rekombinante Protein ein Inhibitor-Ligand von HB-EGF ist.

2. Protein nach Anspruch 1, welches wenigstens die Substitutionen Y380K, Q387E und L390T aufweist.

3. Protein nach Anspruch 1 oder 2, welches zusätzlich die Substitution A395T aufweist.

4. Protein nach einem der Ansprüche 1 bis 3, welches auch wenigstens eine der Substitutionen F389Y und/oder G510A aufweist.

5. Protein nach einem der Ansprüche 1 bis 4, welches auch wenigstens eine Substitution aufweist, welche gewählt ist aus der Gruppe, gebildet aus: N399K, V452T, T517E, V483Q, H492E, S494K, T436H und E497D.

6. Protein nach Anspruch 5, welches die Substitutionen N399K, V452T, T517E, V483Q, H492E und S494K aufweist.

7. Protein nach einem der Ansprüche 1 bis 6, welches eine der Aminosäuresequenzen SEQ ID NR. 2 bis 5 und SEQ ID NR. 7 bis 9 umfasst oder daraus besteht.

8. Protein nach einem der Ansprüche 1 bis 7, welches mittels eines detektierbaren Markers markiert ist.

9. Isoliertes Polynukleotid, welches für ein rekombinantes Protein nach einem der Ansprüche 1 bis 8 kodiert.

10. Rekombinanter Vektor für das Klonen und/oder Exprimieren, welcher ein Polynukleotid nach Anspruch 9 umfasst.

11. Mittels eines Vektors nach Anspruch 10 modifizierte Zelle.

12. Pharmazeutische Zusammensetzung, welche wenigstens ein rekombinantes Protein nach einem der Ansprüche 1 bis 7 umfasst oder einen rekombinanten Expressionsvektor nach Anspruch 10 und einen pharmazeutisch zulässigen Träger.

13. Rekombinantes Protein nach einem der Ansprüche 1 bis 7 oder rekombinanter Expressionsvektor nach Anspruch 10 zur Verwendung in der Behandlung von Erkrankungen im Zusammenhang mit einer Aktivierung des Weges HB-EGF/EGFR, gewählt aus der Gruppe, gebildet aus: rasch fortschreitende Glomerulonephritiden, Krebserkrankungen, Vasospasmus im Zusammenhang mit Gehirnprellungen, kardiale Hypertrohie, Hyperplasie der glatten Muskelzellen, pulmonale Hypertonie, diabetische Retinopathien und arterielle Restenose.

14. Verwendung eines markierten Proteins nach Anspruch 8 zur in vitro-Diagnose einer Erkrankung im Zusammenhang mit einer Aktivierung des Weges HB-EGF/EGFR, gewählt aus der Gruppe, gebildet aus: rasch fortschreitende Glomerulonephritiden, Krebserkrankungen, Vasospasmus im Zusammenhang mit Gehirnprellungen, kardiale Hypertrohie, Hyperplasie der glatten Muskelzellen, pulmonale Hypertonie, diabetische Retinopathien und arterielle Restenose.

## Claims

1. A recombinant protein, comprising an isolated R domain of the diphtheria toxin comprising an amino acid sequence having at least 70% identity with residues 380 to 535 of the amino acid sequence of SEQ ID NO: 1, said sequence comprising the combination of the following substitutions:
- Y380K and L390T,
and said sequence lacking at the N- or C-terminal of said R domain, the sequence of the C domain and the T domain of the diphtheria toxin and the sequence of a protein or a protein domain capable of improving the stability or purification of said R domain, and said recombinant protein being an inhibitory ligand of HB-EGF.

2. A protein according to claim 1, comprising at least the Y380K, Q387E and L390T substitutions.

3. A protein according to claim 1 or 2, further comprising the A395T substitution.

4. A protein according to any of claims 1 to 3, also comprising at least one of the F389Y and/or G510A substitutions.

5. A protein according to any one of claims 1 to 4, also comprising at least one substitution selected from the group consisting of: N399K, V452T, T517E, V483Q, H492E, S494K, T436H and E497D.

6. A protein according to claim 5, comprising the N399K, V452T, T517E, V483Q, H492E and S494 substitutions.

7. A protein according to any one of claims 1 to 6, comprising or consisting of one of the amino acid sequences SEQ ID NO: 2 to 5 and SEQ ID NO: 7 to 9.

8. A protein according to any one of claims 1 to 7, labelled with a detectable marker.

9. An isolated polynucleotide encoding a recombinant protein according to any one of claims 1 to 8.

10. A recombinant cloning and/or expression vector comprising a polynucleotide according to claim 9.

11. A cell modified by a vector according to claim 10.

12. A pharmaceutical composition, comprising at least one recombinant protein according to any one of claims 1 to 7 or a recombinant expression vector of claim 10, and a pharmaceutically acceptable carrier.

13. A recombinant protein according to any one of claims 1 to 7 or the recombinant expression vector according to claim 10, for use in the treatment of diseases associated with pathway activation HB-EGF/EGFR selected from the group consisting of: rapidly progressive glomerulonephritis, cancers, vasospasm associated with brain contusions, cardiac hypertrophy, hyperplasia of smooth muscle cells, pulmonary hypertension, diabetic retinopathies and arterial restenosis.

14. The use of a labelled protein according to claim 8 for the *in vitro* diagnosis of a disease related to an activation of the HB-EGF/EGFR pathway selected from the group consisting of: rapidly progressive glomerulonephritis, cancers, vasospasm associated with brain contusions, cardiac hypertrophy, hyperplasia of smooth muscle cells, pulmonary hypertension, diabetic retinopathies and arterial restenosis.
